(19) Europäisches Patentamt
European Patent Office
Office européen des brevets

(11) Numéro de publication : **0 459 890 B1**

(12) # FASCICULE DE BREVET EUROPEEN

(45) Date de publication du fascicule du brevet : **05.01.94 Bulletin 94/01**

(21) Numéro de dépôt : **91401372.7**

(22) Date de dépôt : **29.05.91**

(51) Int. Cl.$^5$ : **A61K 7/06,** A61K 31/505, C07D 239/34, C07D 239/38, C07D 239/42, C07D 239/46, C07D 239/48, C07D 498/04

(54) **Composition destinée à être utilisée pour freiner la chute des cheveux et pour induire et stimuler leur croissance,contenant des dérivés d'alkyl-2 amino-4 (ou dialkyl-2,4) pyrimidine oxyde-3 et nouveaux composés dérivés d'alkyl-2 amino-4 pyrimidine oxyde-3.**

(30) Priorité : **30.05.90 FR 9006693**

(43) Date de publication de la demande : **04.12.91 Bulletin 91/49**

(45) Mention de la délivrance du brevet : **05.01.94 Bulletin 94/01**

(84) Etats contractants désignés : **AT BE CH DE DK FR GB IT LI NL SE**

(56) Documents cités :
**EP-A- 0 015 365**
**EP-A- 0 303 871**
**EP-A- 0 356 271**
**EP-A- 0 384 370**
**WO-A-85/04577**
**WO-A-86/04231**
**GB-A- 1 143 167**
**US-A- 4 885 296**

(73) Titulaire : **L'OREAL**
**14, Rue Royale**
**F-75008 Paris (FR)**

(72) Inventeur : **Dufetel, Didier**
**Décédé (FR)**
Inventeur : **Estradier, Françoise**
**4, rue Nobel**
**F-75018 Paris (FR)**
Inventeur : **Hocquaux, Michel**
**70, rue du Rendez-vous**
**F-75012 Paris (FR)**

(74) Mandataire : **Casalonga, Axel et al**
**BUREAU D.A. CASALONGA - JOSSE**
**Morassistrasse 8**
**D-80469 München (DE)**

**EP 0 459 890 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne des compositions destinées à être utilisées, notamment en application topique, pour freiner la chute des cheveux et pour induire et stimuler leur croissance, contenant des dérivés d'alkyl-2 amino-4 (ou dialkyl-2,4) pyrimidine oxyde-3, ainsi que des nouveaux dérivés d'alkyl-2 amino-4 pyrimidine oxyde-3 utilisés dans ces compositions.

On connaît déjà, dans l'état de la technique, le diamino-2, 4 pipéridino-6 pyrimidine oxyde-3 ou "Minoxidil" pour ses propriétés d'agent anti-hypertenseur, mais également pour son utilisation dans le traitement de la chute des cheveux, de la pelade, de la dermatite desquamante et de l'alopécie.

La demanderesse a découvert de nouvelles compositions pour le traitement et la prévention de la chute des cheveux, utilisées notamment en application topique, contenant une famille particulière de composés dérivés d'alkyl-2 amino-4 pyrimidine oxyde-3 ou de dialkyl-2,4 pyrimidine oxyde-3.

Les composés retenus par la demanderesse sont efficaces pour la repousse des cheveux et notamment pour induire et stimuler leur croissance et freiner leur chute, et contrairement au Minoxidil, présentent une activité anti-hypertensive sensiblement nulle ou plus faible.

De plus, ces composés présentent des solubilités dans des milieux habituellement utilisés en cosmétique et en pharmacie, nettement supérieures à celles du Minoxidil.

L'invention a donc pour objet de nouvelles compositions destinées au traitement et à la prévention de la chute des cheveux, contenant des composés particuliers dérivés d'alkyl-2 amino-4 (ou dialkyl-2,4) pyrimidine oxyde-3.

L'invention a également pour objet de nouveaux dérivés d'alkyl-2 amino-4 pyrimidine oxyde-3 utilisés dans ces compositions.

Un autre objet concerne l'utilisation des composés selon l'invention pour la préparation d'un médicament destiné au traitement thérapeutique de la chute des cheveux.

D'autres objets de l'invention apparaîtront à la lecture de la description et des exemples qui suivent.

Les compositions conformes à l'invention sont essentiellement caractérisées par le fait qu'elles contiennent dans un milieu physiologiquement acceptable, au moins un composé répondant à la formule suivante :

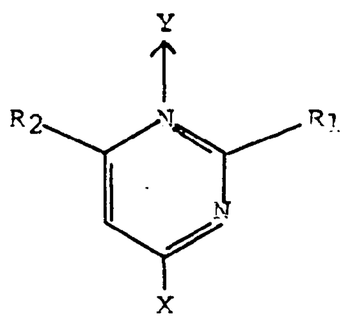

(I)

dans laquelle :

$R_1$ désigne un atome d'hydrogène ou un radical alkyle linéaire saturé en $C_1$-$C_8$;

$R_2$ désigne un radical alkyle linéaire saturé en $C_1$-$C_8$, un groupement -$NRH_3$ dans lequel $R_3$ désigne un atome d'hydrogène ou le groupe -$COOR_4$ où $R_4$ représente un radical alkyle linéaire en $C_1$-$C_4$;

X désigne :

(i) un groupement

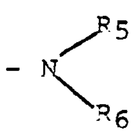

dans lequel :

$R_5$ et $R_6$, identiques ou différents, désignent un atome d'hydrogène, un groupe alkyle en $C_1$-$C_{12}$, linéaire ou ramifié, pouvant être substitué par un ou plusieurs atomes d'halogène, un groupe alcényle linéaire en $C_2$-$C_{12}$, un groupe cycloalkyle en $C_3$-$C_{10}$ ou bien encore un groupe aryle ou aralkyle répondant à la formule :

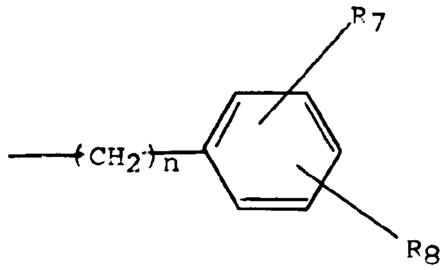

où

n vaut O à 4;

$R_7$ et/ou $R_8$, indépendamment l'un de l'autre, désignent un atome d'hydrogène, un groupement alkyle ou alcoxy inférieur en $C_1$-$C_6$ ou un radical trifluorométhyle;

$R_5$ et $R_6$ peuvent former avec l'atome d'azote auquel ils sont reliés, un hétérocycle saturé ou insaturé choisi parmi les groupes suivants : aziridino, azétidino, pyrrolidino, pipéridino, hexaméthylèneimino, heptaméthylèneimino, octaméthylèneimino, tétrahydropyridino, dihydropyridino, pyrrole, pyrrazole, imidazole, triazole, alkyl-4 pipérazino, morpholino, thiamorpholino;

(ii) un groupement $-OR_9$, dans lequel $R_9$ désigne un radical alkyle en $C_1$-$C_{12}$, linéaire ou ramifié, pouvant être substitué par un ou plusieurs atomes d'halogène, un radical alcényle en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un radical alkyle en $C_1$-$C_6$, un radical alcoxy en $C_1$-$C_6$, un atome d'halogène ou un radical trifluorométhyle;

(iii) un groupement $-SR_{10}$, dans lequel $R_{10}$ a la même signification que le radical $R_9$ défini précédemment;

Y désigne un atome d'oxygène ou un groupement $-OSO_3^{\ominus}$.

Parmi les composés de formule générale (I), selon la présente invention, dans laquelle X désigne un groupement

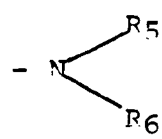

les composés particulièrement préférés sont ceux pour lesquels :

- $R_2$ désigne un radical méthyle et X désigne le groupe pipéridino; ou bien
- $R_2$ désigne un groupement $-NHR_3$, dans lequel $R_3$ a la signification mentionnée dans la définition de la formule (I), et X désigne l'un des groupes amino suivants : diméthylamino, diéthylamino, n-butylamino, pipéridino, morpholino, méthyl-4 pipérazino, benzylamino ou anilino.

Parmi les composés de formule générale (I), selon la présente invention, dans laquelle X désigne le groupement $-OR_9$, les composés particulièrement préférés sont choisis parmi ceux pour lesquels :

- $R_2$ désigne un radical méthyle et X désigne le groupe éthoxy; ou bien
- $R_2$ désigne le groupement $-NHR_3$, dans lequel $R_3$ a la signification indiquée dans la formule (I) et X désigne les groupes alcoxy suivants : éthoxy, butoxy, méthyl-1 éthyloxy et diméthyl-2,4 phényloxy.

Lorsque Y désigne un atome d'oxygène et $R_2$ désigne le groupement $-NHR_3$, les composés de formule (I) existent sous deux formes tautomères, suivant l'équilibre ci-dessous :

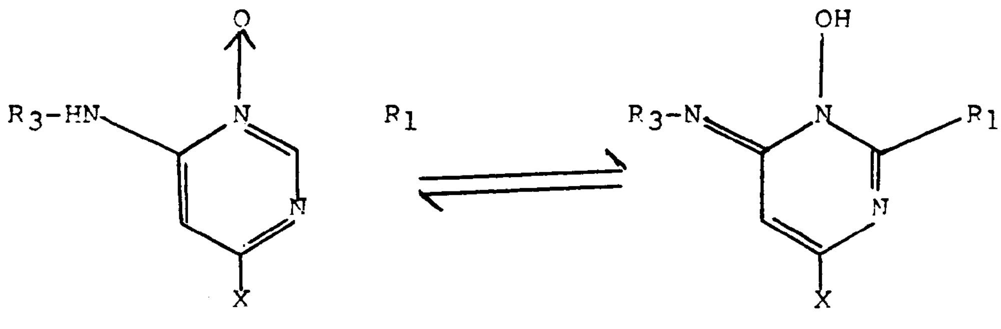

(I) (I2)

Selon la nature du milieu, l'une des formes peut être prépondérante par rapport à l'autre.

Les composés de formule (I), conformes à la présente invention, peuvent être transformés en leurs sels d'addition d'acides cosmétiquement ou pharmaceutiquement acceptables, tels que les sels des acides sulfurique, chlorhydrique, bromhydrique, phosphorique, acétique, benzoïque, salicylique, glycolique, succinique, nicotinique, tartrique, maléique, pamoïque, méthane sulfonique, picrique, lactique, etc...

Parmi les composés de formule générale (I), un certain nombre de composés sont connus en eux-mêmes et ont été décrits comme agents anti-hypertenseurs ou comme intermédiaires de synthèse.

Ils sont notamment décrits dans les brevets américains US-P-3 464 987 et 4 287 338; ou cités dans la littérature technique (Chem. Pharm. Bull. 29 (1), 98-104 (1981)).

Les composés nouveaux qui constituent un autre objet de l'invention, répondent à la formule (I') suivante :

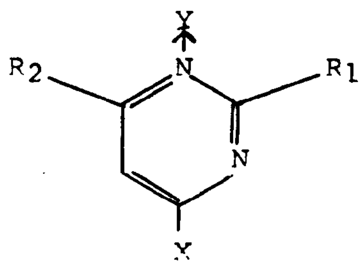

(I')

dans laquelle X, Y, $R_2$, $R_1$ ont les mêmes significations que celles indiquées dans la formule (I) ci-dessus, sous réserve que :

1°) lorsque X désigne

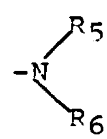,

dans laquelle $R_5$ et $R_6$ ont la même signification indiquée dans la formule (I);

- si Y désigne O, $R_1$ et $R_2$ désignent, indépendamment l'un de l'autre, un groupe alkyle linéaire saturé en $C_1$-$C_8$;
- si Y désigne $OSO_3^{\ominus}$, $R_2$ ne désigne pas $NH_2$;

2°) lorsque X désigne $OR_9$, dans laquelle $R_9$ a la même signification que celle indiquée ci-dessus, si Y désigne O, $R_2$ désigne le groupe $NHR_3$ tel que défini ci-dessus.

Les composés nouveaux de formule (I') peuvent se trouver sous forme de sels d'addition d'acides physiologiquement acceptables.

Les composés conformes à la présente invention répondant à la formule générale (I), sont obtenus à partir d'un dérivé du pyrimidine oxyde-3 substitué en position 6, de formule suivante (II) :

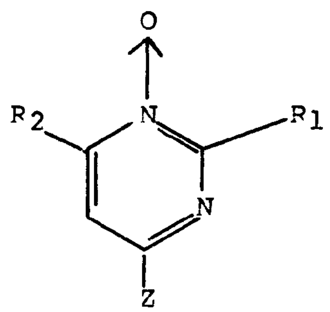

(II)

dans laquelle :

$R_1$ désigne un atome d'hydrogène ou un radical alkyle linéaire saturé en $C_1$-$C_8$;

$R_2$ désigne un groupe amino ou un radical alkyle linéaire saturé en $C_1$-$C_8$;

Z désigne un atome d'halogène choisi parmi le chlore ou le brome, un groupement sulfonate tel que tosylate, brosylate ou mésylate ou un groupement phénoxy substitué par des groupes électro-attracteurs comme les atomes d'halogène ou des groupements nitro.

Les composés de formule (II), suivant la nature du groupe Z, peuvent être synthétisés selon le schéma réactionnel suivant :

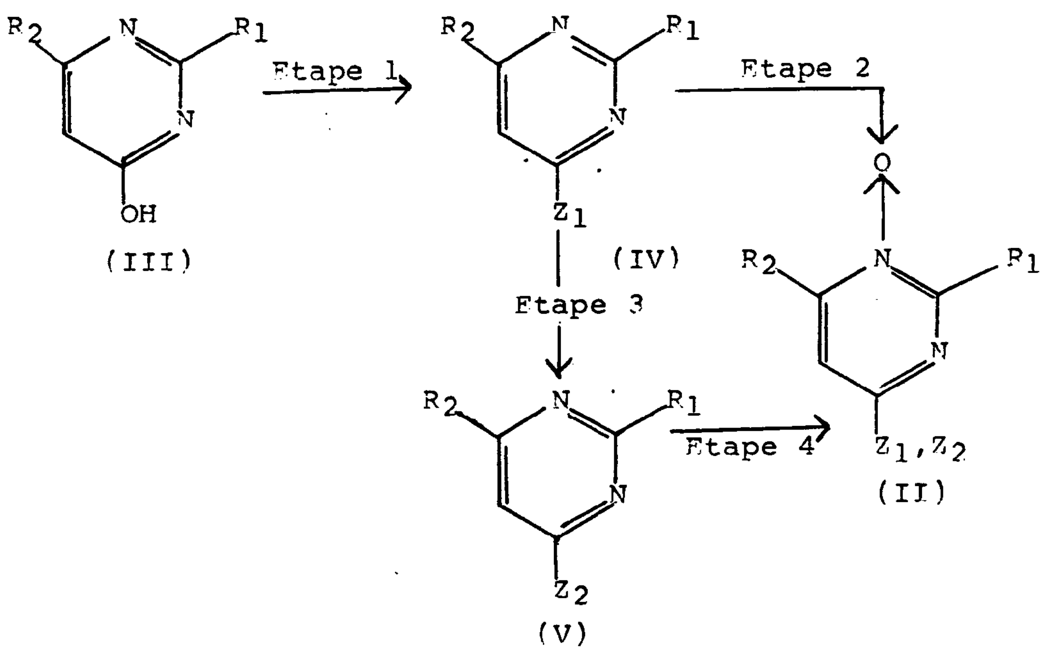


(III) (IV) (V) (II)

Etape 1 :

Les composés de formule (III) hydroxylés en position 6 sont transformés en leurs dérivés halogénés ou sulfonates de formule (IV) dans laquelle $Z_1$ désigne un atome d'halogène ou un groupement sulfonate.

Les méthodes d'halogénation sont classiques et décrites dans la littérature technique (JERRY MARCH, Advanced Organic Chemistry, 3ème édition, page 593); l'agent halogénant le plus utilisé est un oxyhalogénure de phosphore tel que l'oxychlorure de phosphore pour la chloruration.

Les méthodes de sulfonation utilisées sont classiques et décrites dans la littérature technique (JERRY MARCH, Advanced Organic Chemistry, 3ème édition, page 444). Elles consistent à faire réagir un halogénure d'acide sulfonique, en présence de base, sur les composés de formule (III).

Etape 2 :

Les composés de formule (IV) sont facilement oxydés en position para du groupement $Z_1$ par action d'un peracide organique tel que l'acide métachloroperbenzoïque, en présence d'un solvant protique ou aprotique

tel que le dichlorométhane.

Etape 3 :

Les composés (IV) peuvent être substitués par des groupes phénoxy $Z_2$ portant des groupes électro-attracteurs, selon les méthodes classiques décrites dans la littérature, notamment le brevet français n° 1 513 739 (en particulier dans le cas où $R_2$ désigne $NH_2$).

Etape 4 :

Les composés (V) résultant de l'étape 3 sont oxydés en position para du groupement $Z_2$ par action d'un peracide organique, selon les méthodes décrites dans le brevet français n° 1 513 739.

Les composés particuliers, selon l'invention, répondant à la formule (IA) :

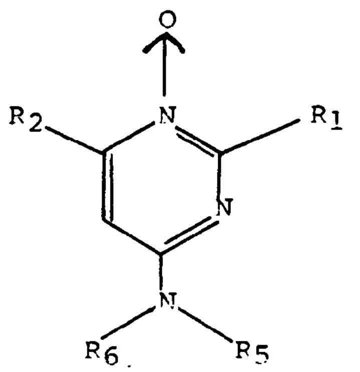

(IA)

dans laquelle $R_1$, $R_2$, $R_5$, $R_6$ ont les significations indiquées dans la formule générale (I) ci-dessus, sont obtenus en faisant réagir une amine

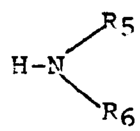

sur les composés de formule (II).

On procède en présence d'un solvant qui peut être un alcool, de préférence l'éthanol, or de l'amine servant de réactif et à la fois de solvant, à une température comprise entre 20 et 150°C, suivant les procédés décrits dans les brevets américains 3 644 364 et 3 464 987 où Z désigne un groupe phénoxy.

La préparation de ces composés peut être représentée par le schéma suivant :

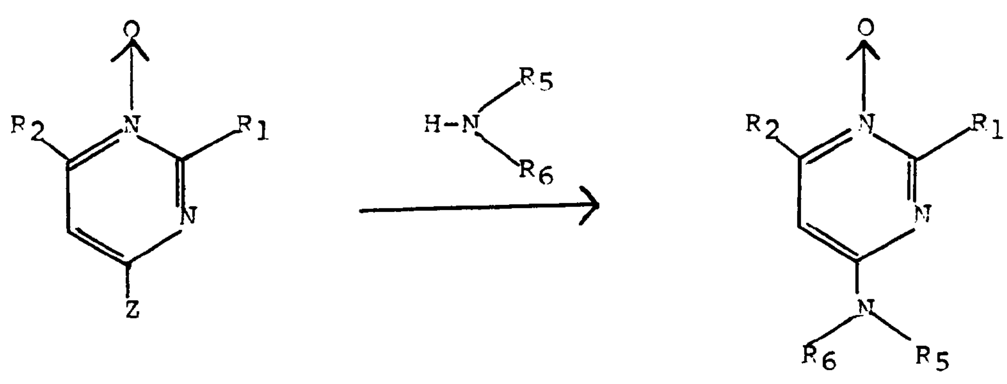

(II) (IA)

SCHEMA A

Les composés particuliers, selon l'invention, de formule (IB) :

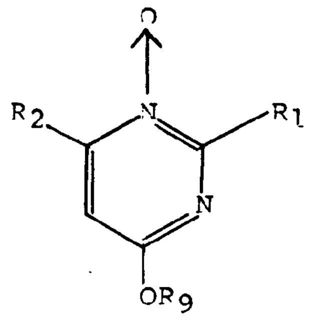

(IB)

sont obtenus en faisant réagir une solution de l'alcoolate $R_9O^{\ominus}$ $W^{\oplus}$ dans laquelle $R_9$ a la même signification indiquée dans la formule (I) et W désigne un métal alcalin tel que le sodium, le potassium ou le lithium, dans l'alcool correspondant, sur les composés de formule (II), dans laquelle Z désigne le chlore ou le brome ou un groupement phénoxy substitué par des groupes électro-attracteurs.

On applique la méthode de Williamson telle que décrite dans le brevet européen EP-57546 à une température comprise entre 40 et 100°C.

La préparation de ces composés peut être représentée par le schéma suivant :

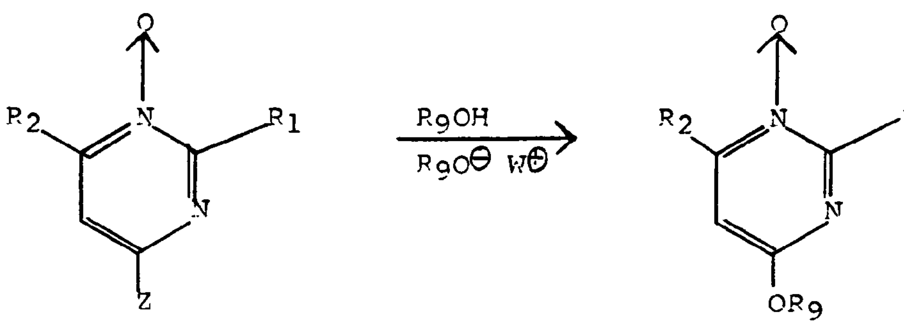


(IB)

SCHEMA B1

Les composés de formule (IB) peuvent être obtenus suivant un autre procédé représenté par le schéma suivant :

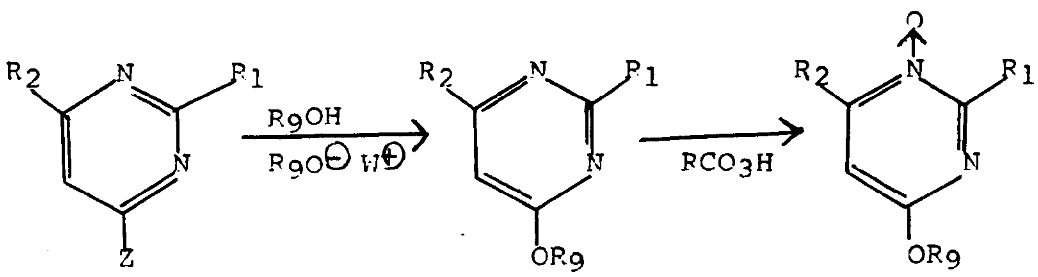


(VI) (VII) (IB)

SCHEMA B2

Ce procédé consiste à effectuer une réaction de Williamson sur le composé de formule (VI), dans laquelle $R_2$, $R_1$ et Z ont les mêmes significations indiquées dans la formule (II), pour obtenir un dérivé alcoxylé en position 6 de formule (VII). Ce dernier est ensuite oxydé sélectivement en position 3 par un peracide organique

doux tel que l'acide métachloroperbenzoïque ou le monoperphtalate de magnésium.

Les composés de formule (IB) sont, d'une manière générale, plus accessibles par la voie du procédé $B_2$, en particulier pour le branchement d'alcoolates stériquement encombrés en position 6, tels que l'isopropylate.

Les composés particuliers, selon l'invention, répondant à la formule (IC) :

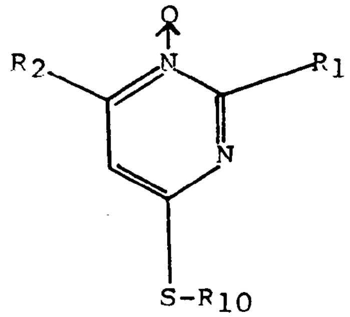

(IC)

dans laquelle $R_1$, $R_2$ et $R_{10}$ ont les significations indiquées précédemment, sont obtenus en faisant réagir sur les composés de formule (II) un thiolate de formule $R_{10}S^{\ominus}W^{\oplus}$, dans laquelle $R_{10}$ et $W^{\oplus}$ ont les mêmes significations que précédemment, en présence d'un solvant choisi parmi les éthers, de préférence le mono-ou diméthyléther de l'éthylèneglycol, à une température de l'ordre de 50 à 150°C.

On procède suivant les méthodes classiques de la littérature (D.J. Brown, The Pyrimidines, Vol.16, Supplément II, Chapitre VI, Section F).

La préparation de ces composés peut être représentée par le schéma C suivant :

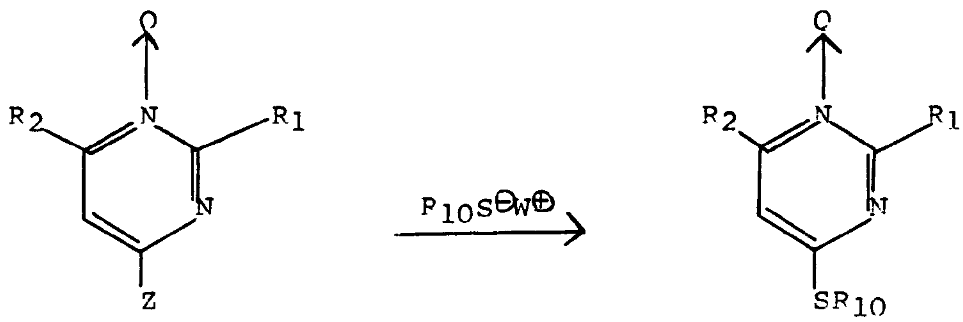


(II) (IC)

SCHEMA C

Les composés particuliers, selon l'invention, de formule (I), dans laquelle Y désigne un atome d'oxygène, obtenus selon les différents procédés décrits précédemment, peuvent être transformés en leurs homologues O-sulfates de formule (ID) définie ci-après, par sulfatation chimique, selon les méthodes classiques décrites dans la littérature (J. Med. Chem., 1983, 26, p. 1791-1793).

On utilise comme réactif de sulfatation les complexes de trioxyde de soufre-pyridine, de trioxyde de soufre-triéthylamine ou de trioxyde de soufre-éthyl diisopropylamine.

Les solvants utilisés sont de préférence le diméthylformamide, l'acétonitrile, le chloroforme ou leurs mélanges binaires. La température est de l'ordre de 0 à 25°C et le temps de réaction varie entre 1 heure et 24 heures.

La préparation des composés de formule (ID) ainsi obtenue peut être représentée par le schéma suivant :

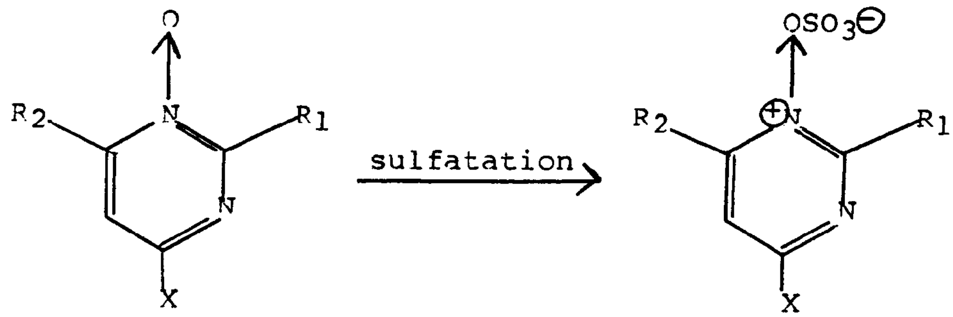


(I) (ID)

SCHEMA D

Les composés de formule générale (I), selon l'invention, dans laquelle Y désigne un atome d'oxygène et $R_2$ le groupe amino $-NH_2$, peuvent être transformés en leurs homologues carbamates de formule (IE) telle que définie ci-dessus.

Les composés (IE) sont préparés selon les méthodes classiques de la littérature (J. March, Advanced Organic Chemistry, 3ème édition, p. 370) par action d'un chloroformiate d'alkyle en présence d'une amine tertiaire telle que la pyridine, comme représenté par le schéma suivant :

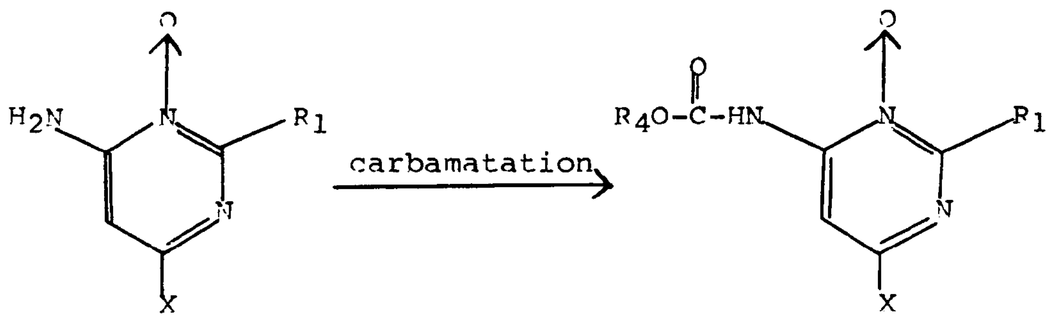


(I) (IE)

SCHEMA E

Les composés de formule (IE) sont facilement hydrolysables en milieu potasse alcoolique, et peuvent redonner naissance à leurs précurseurs de formule (I), dans laquelle Y désigne un atome d'oxygène et $R_2$ un groupe amino $-NH_2$.

Les composés de formule (IE), conformes à l'invention, peuvent constituer des intermédiaires pour la synthèse des oxadiazolopyrimidinones ayant la formule (VIII) définie ci-dessous :

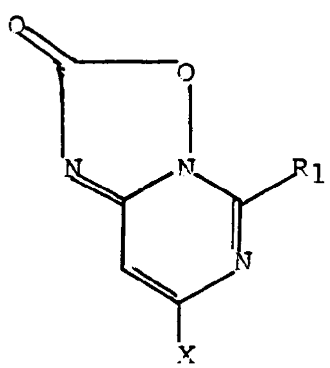

(VIII)

dans laquelle $R_1$ et X ont la signification indiquée dans la formule générale (I).

Les composés (VIII) sont obtenus par cyclisation-élimination interne des dérivés carbamates de formule (IE), selon les méthodes décrites dans la littérature (J.C. MULLER, Helvetica Chimica Acta, Vol. 66, 1983, p. 669-672).

Les composés de formule (VIII) et leurs sels d'addition d'acides cosmétiquement et pharmaceutiquement acceptables sont nouveaux et constituent un autre objet de l'invention. Ils peuvent recevoir des applications diverses et notamment dans l'utilisation pour le traitement et la prévention de la chute des cheveux et la stimulation de leur repousse.

Les compositions conformes à la présente invention, contenant dans un milieu physiologiquement acceptable, au moins un composé répondant à la formule (I) ou un de ses sels d'addition d'acides physiologiquement acceptables, peuvent être appliquées dans le domaine cosmétique ou pharmaceutique, notamment en application topique. Elles sont destinées pour le traitement et la prévention de la chute des cheveux et notamment de la pelade, de l'alopécie ainsi que des dermatites desquamantes et la stimulation de leur repousse.

Ces compositions peuvent comporter, à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou de dispersion vésiculaire, de lotion, de gel, de spray ou de suspension. Elles peuvent être, soit anhydres, soit aqueuses, selon l'indication clinique.

Les composés selon l'invention sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de savon, de shampooing, d'aérosol ou de mousse et contiennent, dans un support cosmétiquement acceptable, au moins un composé de formule (I) ou l'un de ses sels d'addition d'acides.

La concentration de ces composés de formule (I) dans ces compositions est, de préférence, comprise entre 0,01 et 5% en poids et en particulier entre 0,05 et 3% en poids.

Les compositions conformes à l'invention peuvent contenir différents additifs habituellement utilisés en cosmétique ou en pharmacie et en particulier des substances actives, telles que des agents hydratants comme la thiamorpholinone et ses dérivés ou l'urée; des agents antiséborrhéiques, tels que la S-carboxyméthylcystéine, la S-benzylcystéamine, et leurs dérivés; la thioxolone.

Les composés conformes à l'invention peuvent être associés à des composés améliorant encore leur activité sur la repousse et/ou sur le freinage de la chute les cheveux, tels que les composés suivants :

- les esters d'acide nicotinique, dont plus particulièrement les nicotinates d'alkyle en $C_1$-$C_6$ et notamment le nicotinate de méthyle ou le nicotinate de benzyle;
- les agents anti-inflammatoires stéroïdiens et non stéroïdiens bien connus dans l'état de la technique et en particulier l'hydrocortisone, ses sels et ses dérivés, l'acide niflumique;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que plus particulièrement la cinnarizine et le diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le Diazoxyde correspondant au méthyl-3 chloro-7 2H benzothiadiazine 1,2,4-dioxyde-1,1; la Spiroxazone ou 7-(acétylthio)-4', 5'-dihydrospiro [androst 4-ène-17,2'-(3'H)furan]-3 one; des phospholipides, tels que la lécithine; les acides linoléique et linolénique; l'acide salicylique et ses dérivés décrits dans le brevet français 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline ou la trihydroxy-1,8,9 anthracène, les caroténoïdes, les acides eicosatétraynoïque-5,8,11,14 ou eicosatriynoïque-5,8,11, leurs esters et amides.

Les composés conformes à l'invention peuvent également être associés à des agents tensio-actifs dont notamment ceux choisis parmi les agents tensio-actifs non ioniques et amphotères.

Parmi les tensio-actifs non ioniques, on citera les polyhydroxypropyléthers décrits notamment dans les brevets français n° 1 477 048; 2 091 516; 2 169 787; 2 328 763; 2 574 786; les alkyl($C_8$-$C_9$)phénols oxyéthylénés comportant de 1 à 100 moles d'oxyde d'éthylène et de préférence 5 à 35 moles d'oxyde d'éthylène; les alkylpolyglycosides de formule :

$$C_nH_{2n+1}(C_6H_{10}O_5)_xH \qquad (A)$$

dans laquelle n varie de 8 à 15 inclus et x de 1 à 10 inclus.

Parmi les agents tensio-actifs amphotères, on citera plus particulièrement les amphocarboxyglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléther d'alkylèneglycol, en particulier d'alkyle en $C_1$-$C_4$, d'alkylène en $C_1$-$C_4$ glycol ou de dialkylèneglycol, de préférence de dialkylène en $C_1$-$C_4$ glycol, d'alcool éthylique et d'eau, le solvant glycolique désignant plus particulièrement les monoéthyléthers de l'éthylène glycol, le monométhyléther du propylèneglycol, le monométhyléther du diéthylèneglycol.

Les composés conformes à l'invention peuvent également être introduits dans des supports gélifiés ou épaissis, tels que des supports essentiellement aqueux gélifiés par des hétérobiopolysaccharides, tels que la gomme de xanthane ou les dérivés de cellulose, des supports hydroalcooliques gélifiés par des polyhydroxy éthylacrylates ou méthacrylates ou des supports essentiellement aqueux épaissis en particulier par des acides polyacryliques réticulés par un agent polyfonctionnel, tel que les Carbopol vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVB, des agents antioxydants, tels que l' $\alpha$-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en $C_1$-$C_4$ comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylèneglycol, le monométhyléther de diéthylèneglycol. Les solvants, lorsqu'ils sont présents, le sont dans des proportions comprises entre 1 et 80% en poids par rapport au poids total de la composition.

Les milieux physiologiquement acceptables peuvent être épaissis à l'aide d'agents épaississants habituellement utilisés en cosmétique ou pharmacie, et on peut plus particulièrement citer les hétérobiopolysaccharides tels que la gomme de xanthane, les scléroglucanes, les dérivés de cellulose comme les éthers de cellulose, les polymères acryliques, réticulés ou non.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition contenant les composés de formule (I) définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisées dans le traitement de la pelade, de la chute des cheveux, des dermatites desquamantes.

Les exemples suivants sont destinés à illustrer l'invention.

EXEMPLES DE PREPARATION

EXEMPLE 1

Méthyl-2 amino-4 pipéridino-6 pyrimidine oxyde-3.

1ère partie :

Préparation de la méthyl-2 amino-4 chloro-6 pyrimidine.

Dans 720 ml d'oxychlorure de phosphore, on ajoute peu à peu, sous bonne agitation, 150 g (1,2 moles) de méthyl-2 amino-4 hydroxy-6 pyrimidine anhydre. On porte au reflux léger pendant 5 heures. On obtient une solution jaune. Après refroidissement à 20°C, on essore le précipité formé. En évaporant presqu'à sec le filtrat, on récupère un résidu huileux. On coule ce résidu huileux, peu à peu, sur 500 g de glace, sous agitation et en refroidissant le récipient dans la glace. Dans la solution obtenue après un certain temps, on introduit, peu à peu, le précipité. L'addition est exothermique et il faut bien refroidir dans un bain de glace. La dissolution est totale. On refroidit la solution à 0°C et on alcalinise à pH 8 par addition d'ammoniaque à 20% (850 ml). La formation devient très épaisse. On essore, lave sur le filtre avec 100 ml de solution aqueuse à 10% de chlorure de sodium, puis avec 200 ml d'eau. On obtient 144,2 g de produit brut que l'on recristallise dans l'acétonitrile. On récupère 97 g (0,676 moles) de produit pur de point de fusion = 191°C. Le rendement en produit recristallisé est de 56,3%.

Analyse élémentaire pour $C_5H_6N_3Cl$ ; PM = 143,5

| | C | H | N | Cl |
|---|---|---|---|---|
| Calculé | 41,81 | 4,18 | 29,27 | 24,74 |
| Trouvé | 41,70 | 4,25 | 29,39 | 24,64 |

Les spectres de masse et de RMN $^{13}C$ sont conformes à la structure attendue.

2ème partie :

Préparation du méthyl-2 amino-4 chloro-6 pyrimidine oxyde-3.

On dissout partiellement 50,23 g (0,35 moles) de méthyl-2 amino-4 chloro-6 pyrimidine dans 700 ml de méthanol. On refroidit à 0°C et sous bonne agitation, on introduit, par petites fractions, 131,7 g (0,42 moles) d'acide m-chloroperbenzoïque à environ 55%, de façon que la température reste inférieure à 5°C. Après la fin d'introduction, on maintient encore 3 heures en dessous de 5°C puis on laisse revenir à température ambiante. Après 4 heures à 20°C, il ne reste plus que des traces de produit initial. On abandonne au freezing pour la nuit. Le lendemain, on essore, lave le précipité avec 30 ml de méthanol froid. On le reprend sous agitation dans 120 ml de méthanol glacé. On essore, sèche. On obtient 27,4 g de produit pur de point de fusion = 191°C.

On récupère un 2ème jet en concentrant les eaux-mères méthanoliques à environ 200 ml. On essore le précipité, on le lave avec 10 ml de méthanol froid, puis on le reprend dans 250 ml d'éther éthylique sous agitation pour éliminer l'acide m-chlorobenzoïque qu'il contient, et enfin dans 25 ml de méthanol froid. On récupère ainsi 5,43 g de produit pur de point de fusion = 191°C. Le rendement global est de 58,8%.

Analyse élémentaire pour $C_5H_6N_3OCl$ ; PM = 159,5.

| | C | H | N | O | Cl |
|---|---|---|---|---|---|
| Calculé avec 0,5 m $H_2O$ | 35,61 | 4,15 | 24,92 | 14,24 | 21,07 |
| Trouvé | 35,15 | 4,26 | 24,82 | 14,88 | 20,80 |

Les spectres de masse et de RMN $^{13}C$ sont conformes à la structure attendue.

3ème partie :

Préparation du méthyl-2 amino-4 pipéridino-6 pyrimidine oxyde-3.

On porte, au reflux léger, pendant 4 heures 30 minutes, sous agitation et sous azote, une solution de 44 g (0,261 moles) de Méthyl-2 amino-4 chloro-6 pyrimidine oxyde-3 dans 352 ml de pipéridine. On obtient une suspension jaune. On refroidit dans un bain de glace sel, on essore, lave le précipité sur le filtre avec 30 ml de pipéridine, puis avec de l'éther éthylique. On le reprend ensuite, sous agitation, deux fois dans 150 ml d'eau glacée. On essore, sèche sous vide à température ambiante. On obtient 42 g de produit sous forme de monohydrate. On le recristallise dans de l'acétonitrile avec 3% d'eau. On récupère, après séchage à 80°C, 37,8 g (0,181 moles) de produit pur anhydre de point de fusion = 200°-200,5°C. Le rendement est de 69,6%.

Analyse élémentaire pour $C_{10}H_{16}N_4O$ ; PM = 208.

| | C | H | N | O |
|---|---|---|---|---|
| Calculé | 57,69 | 7,69 | 26,92 | 7,69 |
| Trouvé | 57,60 | 7,79 | 26,93 | 7,89 |

Les spectres de masse et de RMN $^{13}C$ sont conformes à la structure attendue.

EXEMPLE 2

Méthyl-2 amino-4 diméthylamino-6 pyrimidine oxyde-3.

MODE OPERATOIRE :

Dans un tricol de 50 ml, muni d'un réfrigérant, d'un thermomètre et d'une agitation magnétique, on place 2 g ($1,254.10^{-2}$ moles) de méthyl-2 amino-4 chloro-6 pyrimidine oxyde-3, préparée suivant la partie 2 de l'exemple 1, en suspension dans 20 ml d'une solution éthanolique de diméthylamine à 33%. On agite le milieu réactionnel pendant 6 heures à 30°C. On refroidit à 5°C puis on ajoute .50 ml de potasse alcoolique à 10%. On agite pendant 1 heure. On filtre sur un verre fritté préalablement rempli de silice. La solution obtenue est évaporée à sec. Le précipité est recristallisé dans un mélange acétonitrile-eau (99,5-0,5). Masse obtenue : 1,56 g.

Rendement : 74%
PF : 215°C

Analyse élémentaire pour $C_7H_{12}N_4O$ ; PM = 168.

| | C | H | N | O |
|---|---|---|---|---|
| Calculé avec 0,2 moles d'eau | 48,95 | 7,22 | 32,63 | 11,18 |
| Trouvé | 48,99 | 7,15 | 32,69 | 10,95 |

Les spectres RMN $^1H$, $^{13}C$ et de masse sont conformes à la structure attendue.

EXEMPLE 3

Méthyl-2 amino-4 diéthylamino-6 pyrimidine oxyde-3.

MODE OPERATOIRE :

Dans un tricol de 50 ml, muni d'un réfrigérant, d'un thermomètre, d'une agitation magnétique et d'une entrée d'argon, on place 3 g ($1,88.10^{-2}$ moles) de méthyl-2 amino-4 chloro-6 pyrimidine oxyde-3, préparée suivant la partie 2 de l'exemple 1, en suspension dans 30 ml de diéthylamine. On agite le milieu réactionnel sous atmosphère d'argon pendant 4 jours à 55°C. On évapore à sec le milieu réactionnel puis on chromatographie le résidu sur gel de silice en utilisant un gradient d'élution acétate d'éthyle/méthanol. Après évaporation, on obtient un produit brut solide qui est recristallisé dans un mélange acétonitrile-eau (99,5-0,5).

Rendement : 15%.

PF : 143°C

Analyse élémentaire pour $C_9H_{16}N_4O$ ; PM = 196.

| | C | H | N | O |
|---|---|---|---|---|
| Calculé avec 0,22 moles d'eau | 54,00 | 8,22 | 28,00 | 9,77 |
| Trouvé | 54,03 | 8,21 | 28,08 | 9,52 |

Les spectres RMN $^1H$ et de masse sont conformes à la structure attendue.

EXEMPLE 4

Méthyl-2 amino-4 morpholino-6 pyrimidine oxyde-3.

Suivant le mode opératoire décrit à l'exemple 3, avec la morpholine.

Température : 90°C

Temps : 4 heures 30 minutes.

Recristallisation dans un mélange acétonitrile-eau (99-1).

Rendement : 23%.

PF : 203°C.

Analyse élémentaire pour $C_9H_{14}N_4O_2$ ; PM = 210.

| | C | H | N | O |
|---|---|---|---|---|
| Calculé avec 0,44 moles d'eau | 49,54 | 6,82 | 25,69 | 17,94 |
| Trouvé | 49,60 | 6,74 | 25,86 | 18,15 |

Les spectres RMN $^1H$ et de masse sont conformes à la structure attendue.

EXEMPLE 5

Méthyl-2 amino-4 (N-méthylpipérazino)-6 pyrimidine oxyde-3.

Suivant le mode opératoire décrit à l'exemple 3, avec la N-méthylpipérazine.
Température : 70°C
Temps : 3 heures 30 minutes.
Recristallisation dans un mélange acétonitrile-eau (97-3).
Rendement : 35%.
PF : 200°C.

Analyse élémentaire pour $C_{10}H_{17}N_5O$ ; PM = 223.

| | C | H | N | O |
|---|---|---|---|---|
| Calculé pour 0,05 moles d'eau | 53,59 | 7,64 | 31,26 | 7,50 |
| Trouvé | 53,59 | 7,66 | 31,43 | 7,29 |

Les spectres RMN $^1H$ et de masse sont conformes à la structure attendue.

EXEMPLE 6

Méthyl-2 amino-4 (n-butylamino)-6 pyrimidine oxyde-3.

MODE OPERATOIRE :

Dans un tricol de 50 ml, muni d'un réfrigérant, d'un thermomètre, d'une agitation magnétique et d'une entrée d'argon, on place 3 g ($1,88.10^{-2}$ moles) de méthyl-2 amino-4 chloro-6 pyrimidine oxyde-3 préparé suivant la partie 2 de l'exemple 1, et 7,53 g (4 eq) de bicarbonate de potassium en suspension dans 30 ml d'éthanol. On agite le milieu réactionnel sous atmosphère d'argon et on introduit 3,7 ml (2 eq) de butylamine. On chauffe à reflux de l'éthanol pendant 24 heures. Le milieu réactionnel est évaporé à sec puis est repris par 20 ml d'eau. On refroidit à 0°C et on filtre sur verre fritté. On obtient 2,6 g de produit brut. Te précipité est dissous dans un mélange acétate d'éthyle-méthanol (80-20). On ajoute 2 g de silice par gramme de produit et on agite pendant 30 minutes. On filtre et on évapore à sec le filtrat. Le précipité est recristallisé dans un mélange acétonitrile-eau (70-30).

Rendement : 21%.
PF : 222°C.

Analyse élémentaire pour $C_9H_{16}N_4O$ ; PM = 196.

| | C | H | N | O |
|---|---|---|---|---|
| Théorie | 55,10 | 8,16 | 28,57 | 8,16 |
| Trouvé | 55,08 | 8,21 | 28,54 | 8,35 |

Les spectres RMN $^1H$ et de masse sont conformes à la structure attendue.

EXEMPLE 7

Méthyl-2 amino-4 benzylamino-6 pyrimidine oxyde-3.

Suivant le mode opératoire décrit à l'exemple 6, avec la benzylamine.
Température : reflux de l'éthanol.
Temps : 27 heures 30 minutes.
Recristallisation dans un mélange acétonitrile-eau (97-3).
Rendement : 21%
PF : 210°C.

Analyse élémentaire pour $C_{12}H_{14}N_4O$ : PM = 230.

| | C | H | N | O |
|---|---|---|---|---|
| Théorie | 62,60 | 6,09 | 24,34 | 6,95 |
| Trouvé | 62,47 | 6,12 | 24,48 | 7,00 |

Les spectres RMN $^1H$ et de masse sont conformes à la structure attendue.

EXEMPLE 8

Méthyl-2 amino-4 anilino-6 pyrimidine oxyde-3.

Suivant le mode opératoire décrit à l'exemple 6, avec l'aniline.
Température : reflux de l'éthanol.
Temps : 3 jours 18 heures.
Recristallisation dans un mélange acétonitrile-eau (99-1).
Rendement : 14%.
PF : 241°C.

Analyse élémentaire pour $C_{11}H_{12}N_4O$ ; PM = 216.

| | C | H | N | O |
|---|---|---|---|---|
| Calculé pour 0,2 moles d'eau | 60,10 | 5,64 | 25,50 | 8,74 |
| Trouvé | 60,06 | 5,75 | 25,47 | 8,44 |

Les spectres RMN $^1H$ et de masse sont conformes à la structure attendue.

EXEMPLE 9

Méthyl-2 Amino-4 éthoxy-6 pyrimidine oxyde-3.

MODE OPERATOIRE :

Dans un tricol de 100 ml, muni d'un réfrigérant, d'un thermomètre, d'une agitation magnétique et d'une entrée d'argon, on dissout 0,48 g de sodium dans 45 ml d'éthanol. On ajoute 3 g ($1,88.10^{-2}$ moles) de méthyl-2 amino-4 chloro-6 pyrimidine oxyde-3, préparé suivant la partie 2 de l'exemple 1, et on porte le milieu réactionnel à reflux pendant 25 heures 30 minutes. On évapore à sec le mélange hétérogène. Le résidu est purifié sur colonne de gel de silice en utilisant un gradient d'élution avec acétate d'éthyleméthanol. Après évaporation, on obtient un produit brut solide qui est recristallisé dans l'acétonitrile.

Rendement : 23%.

PF : 157°C.

Analyse élémentaire pour $C_7H_{11}N_3O_2$ ; PM = 169.

| | C | H | N | O |
|---|---|---|---|---|
| Théorie | 49,70 | 6,51 | 24,85 | 18,93 |
| Trouvé | 49,64 | 6,57 | 24,95 | 18,94 |

Les spectres RMN $^1H$, 13C et de masse sont conformes à la structure attendue.

EXEMPLE 10

Méthyl-2 amino-4 (diméthyl-2,4 phényl)oxy-6 pyrimidine oxyde-3.

Suivant le mode opératoire décrit à l'exemple 9, avec le diméthyl-2,4 phénol.

Température : 75°C.

Temps : 24 heures.

Recristallisation dans l'acétonitrile.

Rendement : 37%.

PF : 159°C.

Analyse élémentaire pour $C_{13}H_{15}N_3O_2$ : PM = 245.

| | C | H | N | O |
|---|---|---|---|---|
| Théorie | 63,67 | 6,12 | 17,14 | 13,06 |
| Trouvé | 63,52 | 6,17 | 17,18 | 13,30 |

Les spectres RMN $^1H$, $^{13}C$ et de masse sont conformes à la structure attendue.

EXEMPLE 11

Méthyl-2 amino-4 (méthyl-1)éthyloxy-6 pyrimidine oxyde-3

1ère partie :

Préparation de la méthyl-2 amino-4 (méthyl-1)éthyloxy-6 pyrimidine.

MODE OPERATOIRE :

Dans un tricol de 250 ml, muni d'un réfrigérant, d'un thermomètre, d'une entrée d'argon et d'une agitation magnétique, on dissout 1,8 g de sodium dans 150 ml d'isopropanol. On ajoute 10 g ($6,97.10^{-2}$ moles) de méthyl-2 amino-4 chloro-6 pyrimidine, préparée suivant la partie 1 de l'exemple 1, et on porte le milieu réactionnel à reflux et sous argon pendant 24 heures. On évapore à sec le mélange hétérogène. Le résidu solide est dissous dans un minimum d'acétate d'éthyle puis est filtré sur un verre fritté préalablement rempli de silice. Le filtrat est ensuite évaporé à sec. Le précipité est repris dans de l'éther de pétrole glacé. On obtient 5,1 g.

Rendement : 44%.

PF : 106°C.

Analyse élémentaire pour $C_8H_{13}N_3O$ ; PM = 167.

| | C | H | N | O |
|---|---|---|---|---|
| Calculé pour 0,08 moles d'eau | 56,97 | 7,81 | 24,92 | 10,28 |
| Trouvé | 57,04 | 7,86 | 25,04 | 10,20 |

Les spectres RMN $^1H$, $^{13}C$ et de masse sont conformes à la structure attendue.

2ème partie :

Préparation de la méthyl-2 amino-4(méthyl-1)éthyloxy-6 pyrimidine oxyde-3.

MODE OPERATOIRE :

Dans un tricol de 50 ml, muni d'un réfrigérant, d'un thermomètre, et d'une agitation magnétique, on place 2 g ($1,2.10^{-2}$ moles) de méthyl-2 amino-4(méthyl-l)éthyloxy-6 pyrimidine en suspension dans 30 ml d'éthanol. On refroidit à 0°C et on ajoute par petites spatulées de l'acide métachloroperbenzoïque (2 éq). On agite pendant 4 heures entre 0 et 5°C. Le milieu réactionnel est purifié sur colonne de gel de silice en utilisant un gradient d'élution avec acétate d'éthyle-méthanol. Après évaporation, on obtient un produit brut solide qui est recristallisé dans du toluène.

Rendement : 65%.

PF : 140°C.

Analyse élémentaire pour $C_8H_{13}N_3O_2$ ; PM = 183.

| | C | H | N | O |
|---|---|---|---|---|
| Calculé pour 0,2 moles d'eau | 51,44 | 7,18 | 22,51 | 18,86 |
| Trouvé | 51,51 | 7,30 | 22,62 | 18,45 |

Les spectres RMN $^1H$, $^{13}C$ et de masse sont conformes à la structure attendue.

EXEMPLE 12

Méthyl-2 amino-4 butoxy-6 pyrimidine oxyde-3.

1ère partie :

Préparation de la méthyl-2 amino-4 butoxy-6 pyrimidine.

Suivant le mode opératoire décrit dans la partie 1 de l'exemple 11, avec le butanol.
Température : 90°C.
Temps : 3 heures.
Recristallisation dans un mélange éther de pétrole-acétone.
Rendement : 69%.
PF : 92°C.

Analyse élémentaire pour $C_9H_{15}N_3O$ ; PM = 181.

| | C | H | N | O |
|---|---|---|---|---|
| Théorie | 59,67 | 8,29 | 23,20 | 8,84 |
| Trouvé | 59,94 | 8,31 | 23,29 | 9,02 |

Les spectres RMN $^1H$ et de masse sont conformes à la structure attendue.

2ème partie :

Préparation de la méthyl-2 amino-4 butoxy-6 pyrimidine oxyde-3.

Suivant le mode opératoire décrit dans la partie 2 de l'exemple 11, avec la méthyl-2 amino-4 butoxy-6 pyrimidine.
Température : 0-10°C.
Temps : 6 heures.
Recristallisation dans le cyclohexane.
Rendement : 46%.
PF : 94°C.

Analyse élémentaire pour $C_9H_{15}N_3O_2$ ; PM = 197.

| | C | H | N | O |
|---|---|---|---|---|
| Calculé pour 0,4 moles d'eau | 52,88 | 7,74 | 20,56 | 18,80 |
| Trouvé | 52,83 | 8,00 | 20,61 | 18,77 |

Les spectres RMN $^1H$, $^{13}C$ et de masse sont conformes à la structure attendue.

EXEMPLE 13

Méthyl-2 éthoxycarbonylamino-4 pipéridino-6 pyrimidine oxyde-3.

MODE OPERATOIRE :

Dans un tricol de 50 ml, muni d'un réfrigérant, d'un thermomètre, d'une agitation magnétique et d'une ampoule à addition, on place 2 g ($9{,}6.10^{-3}$ moles) de méthyl-2 amino-4 pipéridino-6 pyrimidine oxyde-3 en suspension dans 20 ml de dichlorométhane préalablement séché sur tamis moléculaire. On ajoute 1,1 équivalent de triéthylamine, soit 1,5 ml. On refroidit le milieu réactionnel à 0°C et on additionne goutte à goutte 1,1 équivalent de chloroformiate d'éthyle, soit 1 ml. On laisse revenir à température ambiante (20°C) et on agite pendant 4 heures. On ajoute 10 ml d'eau au milieu réactionnel. La phase organique est extraite par :

- 10 ml d'acide chlorhydrique 1%.
- 10 ml d'une solution de carbonate de sodium 1%.
- 2 x 10 ml d'eau.

puis séchée sur sulfate de sodium. Après filtration sur papier, la solution est évaporée. On obtient un précipité blanc. Masse obtenue = 1,8 g. Ce précipité est recristallisé dans 9 ml d'eau. Masse obtenue : 1,4 g.

Rendement : 52%.

PF : 134°C.

Analyse élémentaire pour $C_{13}H_{20}N_4O_3$ : PM = 280.

| | C | H | N | O |
|---|---|---|---|---|
| Théorie | 55,71 | 7,14 | 20,00 | 17,14 |
| Trouvé | 55,75 | 7,21 | 20,16 | 17,34 |

Les spectres RMN $^1H$, $^{13}C$ et de masse sont conformes à la structure attendue.

EXEMPLE 14

Sel interne de l'hydroxyde du méthyl-2 amino-4 pipéridino-6 sulfoxy-3 pyrimidinium.

1ère méthode :

A une solution de .3,42 ml (0,02 moles) de N,N-diisopropyléthylamine dans 25 ml de chloroforme refroidie dans la glace, est ajouté, sous agitation, 0,666 ml (0,01 moles) d'acide chlorosulfonique. Après une attente de 30 minutes, on ajoute 1,04 g (0,005 moles) de méthyl-2 amino-4 pipéridino-6 pyrimidine oxyde-3 préparé selon l'exemple 1, et l'on maintient pendant 4 heures 30 minutes à 15°-20°C sous azote. Le solvant est évaporé. Le résidu, repris dans un peu d'eau, laisse cristalliser un produit blanc que l'on filtre. Après recristalli-sation dans un mélange DMF-eau, on obtient 1,3 g de sel interne pur qui se décompose à 235°C. Le rendement est de 90,2%.

Analyse élémentaire pour $C_{10}H_{16}N_4O_4S$ ; PM = 288.

| | C | H | N | O | S |
|---|---|---|---|---|---|
| Calculé | 41,66 | 5,55 | 19,45 | 22,22 | 11,11 |
| Trouvé | 41,49 | 5,59 | 19,46 | 22,11 | 11,08 |

Les spectres de masse et RMN $^1H$ sont conformes à la structure attendue.

2ème méthode :

Dans une suspension de 1,04 g (0,005 moles) de méthyl-2 amino-4 pipéridino-6 pyrimidine oxyde-3 dans 15 ml de DMF, est ajouté sous agitation 3,18 g (0,02 moles) de complexe de trioxyde de soufre-pyridine. On maintient pendant 6 heures à températuure ambiante. On dilue la solution obtenue par 60 g d'eau glacée. On filtre le précipité blanc obtenu et on le lave à l'eau glacée. Après recristallisation dans un mélange DMF-eau, on obtient 1,1 g de sel interne pur qui se décompose à 235°C. Le rendement est de 76,4%.

EXEMPLE 15

Sel interne de l'hydroxyde du méthyl-2 amino-4 diméthylamino-6 sulfoxy-3 pyrimidinium.

A une solution de 0,72 ml (0,0042 moles) de N,N-diisopropyléthylamine dans 12 ml d'un mélange chloroforme-diméthylformamide 1-1, refroidie dans la glace, est ajouté, sous agitation, 0,133 ml (0,002 moles) d'acide chlorosulfonique. Après attente de 30 minutes, on ajoute 0,168 g (0,001 moles) de méthyl-2 amino-4 diméthylamino-6 pyrimidine oxyde-3, préparé selon l'exemple 2 et l'on maintient pendant 7 heures entre 5° et 10°C sous azote. Le solvant est évaporé. Le résidu, repris dans un peu d'eau, laisse cristalliser un produit blanc que l'on filtre. Après recristallisation dans un mélange DMF-eau, on obtient 0,11 g de sel interne pur qui se décompose à 226°C. Le rendement est de 44,3%.

Analyse élémentaire pour $C_7H_{12}N_4O_4S$ ; PM = 248.

| | C | H | N | O | S |
|---|---|---|---|---|---|
| Calculé | 33,87 | 4,84 | 22,58 | 25,81 | 12,90 |
| Trouvé | 33,90 | 4,90 | 22,67 | 26,00 | 12,84 |

Les spectres de masse et RMN $^1H$ sont conformes à la structure attendue.

EXEMPLE 16

Diméthyl-2,4 pipéridino-6 pyrimidine oxyde-3.

1ère partie :

Préparation de la diméthyl-2,4 chloro-6 pyrimidine.

MODE OPERATOIRE :

Dans un tricol de 250 ml, muni d'un thermomètre, d'un réfrigérant et d'une agitation magnétique, on introduit 20 g ($16,1.10^{-2}$moles) de diméthyl-2,4 hydroxy-6 pyrimidine en suspension dans 150 ml d'oxychlorure de phosphore. On chauffe à 90°C pendant 1 heure 1/2. Le milieu est ensuite refroidi puis évaporé à sec. Le résidu qui cristallise à température ambiante est ajouté, par petites portions, à un mélange binaire, constitué de dichlorométhane (200 ml) et d'ammoniaque à 10% (300 ml), préalablement refroidi à -5°C dans un bain glacé contenant du chlorure de sodium. La mixture est agitée pendant 1/2 heure à froid puis la phase organique est décantée, lavée à l'eau jusqu'à neutralité puis séchée sur sulfate de sodium, filtrée et évaporée. Le liquide jaune obtenu est filtré sur un verre fritté rempli de silice avec le système éluant dichlorométhaneméthanol (99/1); les fractions pures recueillies sont évaporées. On obtient 16,4 g d'un liquide incolore à odeur persistante et qui cristallise au réfrigérateur.

Rendement : 71%.

Analyse élémentaire pour $C_6H_7ClN_2$ ; PM = 142,5.

| | C | H | N | Cl |
|---|---|---|---|---|
| Calculé | 50,53 | 4,91 | 19,65 | 24,91 |
| Trouvé | 50,61 | 4,88 | 19,55 | 24,94 |

Les spectres RMN $^1H$, $^{13}C$, et de masse sont conformes à la structure attendue.

2ème partie :

Préparation de la diméthyl-2,4 chloro-6 pyrimidine oxyde-3.

Dans un tricol de 500 ml, muni d'un thermomètre et d'une agitation magnétique, on solubilise 20 g ($14,03.10^{-2}$ moles) de diméthyl-2,4 chloro-6 pyrimidine dans 300 ml de dichlorométhane. On refroidit à 5°C puis on ajoute, par portions solides, 65,3 g d'acide métachloroperbenzoïque à 55%, soit environ 1,5 équivalent. Le milieu réactionnel est alors agité pendant 24 heures à température ambiante (25°C) puis filtré sur fritté 3. Le filtrat est lavé par 150 ml d'une solution aqueuse de soude à 6% puis par 2 x 50 ml d'eau pour revenir à neutralité. Il est ensuite séché sur sulfate de sodium puis filtré sur papier et évaporé. La masse brute est recristallisée dans le n-hexane.

Masse obtenue : 10,89 g.
Rendement : 49%.
PF : 100°C.

Analyse élémentaire pour $C_6H_7N_2OCl$; PM = 158.

| | C | H | N | O | Cl |
|---|---|---|---|---|---|
| Calculé avec 0,8 mole $H_2O$ | 45,00 | 4,48 | 17,50 | 10,33 | 22,13 |
| Trouvé | 45,07 | 4,42 | 17,59 | 10,97 | 22,15 |

Le spectre RMN $^{13}C$ et le spectre de masse sont conformes à la structure attendue.

3ème partie :

Préparation de la diméthyl-2,4 pipéridino-6 pyrimidine oxyde-3.

Dans un tricol de 250 ml, muni d'un thermomètre, d'un réfrigérant et d'une agitation magnétique, on introduit 100 ml de pipéridine puis on additionne, par portions solides, 13,5 g ($8,51.10^{-2}$ moles) de diméthyl-2,4 chloro-6 pyrimidine oxyde-3, de telle sorte que l'exotherme ne se développe pas au-delà de 60°C. On laisse ensuite revenir le milieu réactionnel à température ambiante; le chlorhydrate de pipéridinium est alors éliminé de la mixture par filtration et le filtrat est évaporé à sec. L'huile brute obtenue cristallise rapidement à l'ambiante. La masse cristalline est dissoute dans un minimum d'éthanol puis addition d'éther éthylique, à froid, jusqu'à précipitation. On agite pendant 1/2 heure puis on filtre le précipité et on l'essore. Une filtration finale sur gel de silice avec le système éluant dichlorométhane 90-méthanol 10 permet l'obtention de cristaux blancs.

Masse obtenue : 6,5 g.
Rendement : 37%.
PF : 137°C.

Analyse élémentaire pour $C_{11}H_{17}N_3O$ ; PM = 207.

| | C | H | N | O |
|---|---|---|---|---|
| Calculé avec | | | | |
| 0,1 mole $H_2O$ | 63,21 | 8,23 | 20,11 | 8,52 |
| Trouvé | 63,19 | 8,20 | 20,01 | 8,57 |

Les spectres RMN $^1H$, $^{13}C$ et de masse sont conformes à la structure attendue.

## EXEMPLE 17

Sel interne de l'hydroxyde du diméthyl-2,4 pipéridino-6 sulfoxy-3 pyrimidinium.

Suivant le mode opératoire de la 1ère méthode décrite à l'exemple 14, avec 1,035 g de diméthyl-2,4 pipéridino-6 pyrimidine oxyde-3 préparé selon l'exemple 16.

Le temps de réaction est de 2 heures 30 minutes et la température est maintenue entre 0°C et 5°C. Le produit obtenu, recristallisé dans un mélange diméthylformamide-eau se décompose à 202°C.

Le rendement est de 69,6%.

Analyse élémentaire pour $C_{11}H_{37}N_3O_4S$ ; PM = 287.

| | C | H | N | O | S |
|---|---|---|---|---|---|
| Calculé avec | | | | | |
| 0,16 m $H_2O$ | 45,53 | 5,97 | 14,49 | 22,96 | 11,04 |
| Trouvé | 45,60 | 5,98 | 14,41 | 22,96 | 11,10 |

Les spectres de masse et de RMN $^1H$ sont conformes à la structure attendue.

## EXEMPLE 18

Diméthyl-2,4 éthoxy-6 pyrimidine oxyde-3.

MODE OPERATOIRE :

Dans un tricol de 50 ml, muni d'un réfrigérant, d'un thermomètre, d'une agitation magnétique et d'une entrée d'argon, on dissout 0,32 g (1,1 éq) de sodium dans 30 ml d'éthanol absolu. On ajoute 2 g (0,0126 moles) de diméthyl-2,4 chloro-6 pyrimidine oxyde-3, préparé suivant la partie 2 de l'exemple 16, et on laisse agiter à température ambiante pendant 2 heures 15 minutes. On neutralise le milieu réactionnel avec de l'éthanol chlorhydrique. On évapore à sec. Le résidu est purifié sur colonne de gel de silice en utilisant comme éluant un mélange acétate d'éthyle-méthanol. Après évaporation, on obtient un produit brut solide qui est recristallisé dans l'hexane.

Rendement : 81%.

PF : 101°C.

Analyse élémentaire pour $C_8H_{12}N_2O_2$ ; PM = 168.

| | C | H | N | O |
|---|---|---|---|---|
| Théorie | 57,14 | 7,14 | 16,67 | 19,05 |
| Trouvé | 57,09 | 7,20 | 16,57 | 19,01 |

Les spectres RMN $^1$H et de masse sont conformes à la structure attendue.

## EXEMPLE DE COMPOSITION 1

On prépare la composition suivante :

| | | | |
|---|---|---|---|
| - Diméthyl-2,4 pipéridino-6 pyrimidine oxyde-3 | | | 5,0 g |
| - Propylèneglycol | | 22,8 g | 95,0 g |
| - Alcool éthylique | | 55,1 g | |
| - Eau | qsp | 100,0 g | |

Cette composition se présente sous forme d'une lotion.

1 à 2 g de cette composition sont appliqués sur les zones alopéciques du cuir chevelu, éventuellement accompagné d'un massage pour favoriser sa pénétration, à raison d'une à deux applications par jour, pendant trois mois de traitement.

## EXEMPLE DE COMPOSITION 2

On prépare la composition suivante :

| | | | |
|---|---|---|---|
| - Méthyl-2 amino-4 pipéridino-6 pyrimidine oxyde-3 | | | 8,5 g |
| - Propylèneglycol | | 22,8 g | 91,5 g |
| - Alcool éthylique | | 55,1 g | |
| - Eau | qsp | 100,0 g | |

Cette composition se présente sous forme d'une lotion.

## EXEMPLE DE COMPOSITION 3

On prépare une lotion de composition suivante :

| | | | |
|---|---|---|---|
| - Méthyl-2 amino-4 pipéridino-6 pyrimidine oxyde-3 | | | 6,0 g |
| - Alcool éthylique | | 50,0 g | 94,0 g |
| - Eau | qsp | 100,0 g | |

1 à 2 ml de ces lotions sont appliqués sur les zones alopéciques du cuir chevelu; ces applications, éventuellement accompagnées par un massage pour favoriser la pénétration, étant effectuées une ou deux fois par jour.

EXEMPLE DE COMPOSITION 4

On prépare la composition suivante :

| | | | |
|---|---|---|---|
| - Méthyl-2 amino-4 pipéridino-6 pyrimidine oxyde-3 | | | 4,0 g |
| - Propylèneglycol | | 6,45 g | 96,0 g |
| - Ethanol absolu | qsp | 100,0 g | |

Cette composition se présente sous forme d'une lotion.

## Revendications

1. Composition destinée à être utilisée pour freiner la chute des cheveux et pour induire et stimuler leur croissance, caractérisée par le fait qu'elle contient dans un milieu physiologiquement acceptable, au moins un composé répondant à la formule suivante (I) :

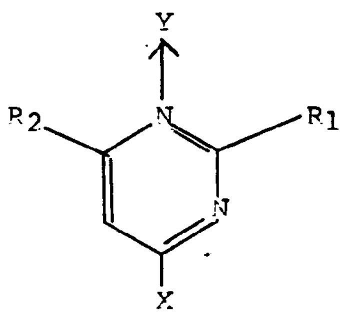

(I)

dans laquelle :

$R_1$ désigne un atome d'hydrogène ou un radical alkyle linéaire saturé en $C_1$-$C_8$;

$R_2$ désigne un radical alkyle linéaire saturé en $C_1$-$C_8$, ou un groupement -$NHR_3$ dans lequel $R_3$ désigne un atome d'hydrogène ou le groupe -$COOR_4$, $R_4$ représentant un radical alkyle linéaire en $C_1$-$C_4$;

X désigne :

i) un groupement

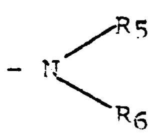

dans lequel :

$R_5$ et $R_6$, identiques ou différents, désignent un atome d'hydrogène, un groupe alkyle en $C_1$-$C_{12}$, linéaire ou ramifié, pouvant être substitué par un ou plusieurs atomes d'halogène, un groupe alcényle linéaire en $C_2$-$C_{12}$, un groupe cycloalkyle en $C_3$-$C_{10}$ ou un groupe aryle ou aralkyle répondant à la formule :

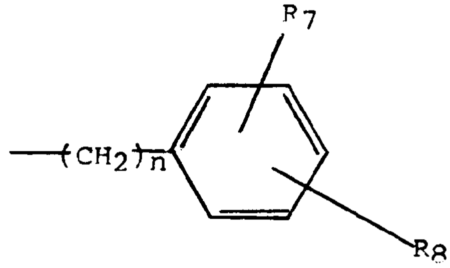

où

n vaut O à 4; et

$R_7$ et/ou $R_8$, indépendamment l'un de l'autre, désignent un atome d'hydrogène, un groupement alkyle ou alcoxy inférieur en $C_1$-$C_6$ ou un radical trifluorométhyle;

$R_5$ et $R_6$ peuvent former avec l'atome d'azote auquel ils sont reliés, un hétérocycle saturé ou insaturé choisi parmi les groupes suivants : aziridino, azétidino, pyrrolidino, pipéridino, hexaméthylèneimino, heptaméthylèneimino, octaméthylèneimino, tétrahydropyridino, dihydropyridino, pyrrole, pyrrazole, imidazole, triazole, alkyl-4 pipérazino, morpholino, thiamorpholino;

(ii) un groupement -$OR_9$, dans lequel $R_9$ désigne un radical alkyle en $C_1$-$C_{12}$, linéaire ou ramifié, pouvant être substitué par un ou plusieurs atomes d'halogène, un radical alcényle en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un radical alkyle en $C_1$-$C_6$, un radical alcoxy en $C_1$-$C_6$, un atome d'halogène ou un radical trifluorométhyle;

(iii) un groupement -$SR_{10}$ dans lequel $R_{10}$ a la même signification que $R_9$ défini ci-dessus;

Y désigne un atome d'oxygène ou un groupement -$OSO_3^{\ominus}$;

ou ses sels d'addition d'acides physiologiquement acceptables.

2. Composition selon la revendication 1, caractérisée par le fait que le composé de formule (I) est choisi parmi ceux pour lesquels :

$R_1$ et $R_2$ désignent un radical méthyle et X désigne le groupe pipéridino; ou bien

$R_1$ désigne méthyle et $R_2$ désigne -$NHR_3$, dans lequel $R_3$ a la même signification indiquée dans la revendication 1, et X désigne diméthylamino, diéthylamino, n-butylamino, pipéridino, morpholino, méthyl-4 pipérazino, benzylamino ou anilino.

3. Composition selon la revendication 1, caractérisée par le fait que le composé de formule (I) est choisi parmi ceux pour lesquels :

$R_1$ et $R_2$ désignent méthyle et X représente le groupe éthoxy; ou bien

$R_1$ désigne méthyle et $R_2$ désigne -$NHR_3$, dans lequel $R_3$ a la même signification mentionnée dans la formule (I) et X désigne le groupe éthoxy, butoxy, méthyl-1 éthyloxy ou diméthyl-2,4 phényloxy.

4. Composition pharmaceutique ou cosmétique, destinée à être utilisée en application topique, caractérisée par le fait qu'elle contient dans un milieu physiologiquement acceptable, au moins un composé tel que défini dans l'une quelconque des revendications 1 à 3.

5. Composition selon la revendication 4, caractérisée par le fait qu'elle se présente sous forme d'onguent, de teinture, de crème, de pommade, de poudre, de timbre, de tampon imbibé, de solution, d'émulsion, de dispersion vésiculaire, de lotion, de gel, de spray ou de suspension anhydre ou aqueuse, en vue de son application pharmaceutique et qu'elle contient au moins un composé tel que défini dans l'une quelconque des revendications 1 à 3.

6. Composition pharmaceutique selon la revendication 5, caractérisée par le fait que le composé de formule (I) est présent dans des concentrations comprises entre 0,1 et 10% en poids par rapport au poids total de la composition.

7. Composition destinée à être utilisée en cosmétique, telle que définie dans la revendication 4, caractérisée par le fait qu'elle se présente sous forme de lotion, de gel, de savon, de shampooing, d'aérosol ou de mousse et qu'elle contient, dans un support cosmétiquement acceptable, au moins un composé tel que défini dans l'une quelconque des revendications 1 à 3, à une concentration comprise entre 0,01 et 5% en

poids.

**8.** Composition selon l'une quelconque des revendications 4 à 7, caractérisée par le fait qu'elle contient en outre des agents hydratants et des agents antiséborrhéiques.

**9.** Composition selon l'une quelconque des revendications 4 à 8, caractérisée par le fait qu'elle contient également des agents améliorant encore l'activité des composés de formule (I) au niveau de la repousse et/ou du freinage de la chute des cheveux.

**10.** Composition selon la revendication 9, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non-stéroïdiens, des rétinoïdes, des agents antibactériens, des agents antagonistes du calcium, des hormones, des agents anti-androgènes, des capteurs de radicaux OH.

**11.** Composition selon la revendication 9, caractérisée par le fait qu'elle contient à titre de composés améliorant encore l'activité sur la repousse et/ou le freinage de la chute des cheveux, des composés choisis parmi le diazoxide, la spiroxazone, les phospholipides, les acides linoléique et linolénique, l'acide salicylique et ses dérivés, les acides hydroxycarboxyliques ou cétocarboxyliques, leurs esters, les lactones et leurs sels correspondants, l'anthraline, les caroténoïdes, les acides eicosatétrainoïque-5,8,11, 14, et eicosatriynoïque-5,8,11, leurs esters et amides.

**12.** Composition selon l'une quelconque des revendications 4 à 11, caractérisée par le fait qu'elle contient également des agents tensio-actifs choisis parmi les agents tensio-actifs non-ioniques et amphotères.

**13.** Composition selon l'une quelconque des revendications 4 à 12, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

**14.** Composition selon la revendication 13, caractérisée par le fait que les solvants sont choisis parmi les alcools inférieurs en $C_1$-$C_4$, les alkylène glycols et les alkyléthers de mono- et de dialkylène glycol.

**15.** Composition selon l'une quelconque des revendications 4 à 14, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UVA et UVB, des agents antioxydants.

**16.** Composé, caractérisé par le fait qu'il répond à la formule suivante :

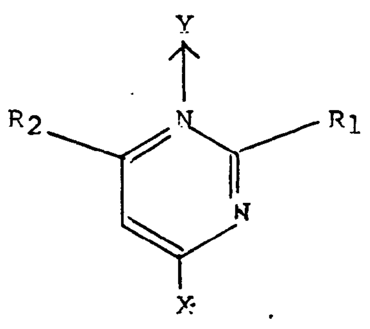

(I')

dans laquelle :

$R_1$ désigne un atome d'hydrogène ou un radical alkyle linéaire saturé en $C_1$-$C_8$;

$R_2$ désigne un radical alkyle linéaire saturé en $C_1$-$C_8$, un groupement -$NHR_3$ dans lequel $R_3$ désigne un atome d'hydrogène ou le groupe -$COOR_4$ où $R_4$ représente un radical alkyle linéaire en $C_1$-$C_4$;

Y désigne un atome d'oxygène ou le groupement $OSO_3^{\ominus}$;

X désigne :

(i) un groupement

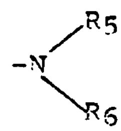

dans laquelle :

$R_5$ et $R_6$, identiques ou différents, désignent un atome d'hydrogène, un groupe alkyle en $C_1$-$C_{12}$, linéaire ou ramifié, pouvant être substitué par un ou plusieurs atomes d'halogène, un groupe alcényle linéaire en $C_1$-$C_{12}$, un groupe cycloalkyle en $C_3$-$C_{10}$ ou un groupe aryle ou aralkyle répondant à la formule :

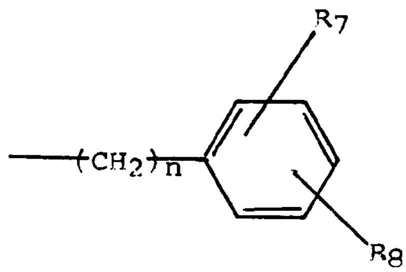

où

n vaut O à 4;

$R_7$ et/ou $R_8$, indépendamment l'un de l'autre, désignent un atome d'hydrogène, un groupement alkyle ou alcoxy inférieur en $C_1$-$C_6$ ou un radical trifluorométhyle;

$R_5$ et $R_6$ peuvent former avec l'atome d'azote auquel ils sont reliés, un hétérocycle saturé ou insaturé choisi parmi les groupes suivants : aziridino, azétidino, pyrrolidino, pipéridino, hexaméthylèneimino, heptaméthylèneimino, octaméthylèneimino, tétrahydropyridino, dihydropyridino, pyrrole, pyrrazole, imidazole, triazole, alkyl-4 pipérazino, morpholino, thiamorpholino; sous réserve que :

- lorsque Y désigne un atome d'oxygène, $R_1$ et $R_2$ désignent, indépendamment l'un de l'autre, un radical alkyle linéaire saturé en $C_1$-$C_8$ et ne désignent pas simultanément le radical méthyle lorsque X désigne diméthylamino ;
- lorsque Y désigne le groupe $OSO_3^{\ominus}$, $R_2$ ne désigne pas $-NH_2$;

(ii) un groupement $-OR_9$, dans lequel $R_9$ désigne un radical alkyle en $C_1$-$C_{12}$, linéaire ou ramifié, pouvant être substitué par un ou plusieurs atomes d'halogène, un radical alcényle en $C_2$-$C_{12}$, un radical cycloalkyle en $C_3$-$C_{10}$, un radical aralkyle en $C_7$-$C_{12}$, sous réserve que :

- lorsque Y désigne un atome d'oxygène, $R_2$ désigne un groupement $-NHR_3$ tel que défini ci-dessus;

(iii) un groupe $-SR_{10}$ dans lequel $R_{10}$ a la même signification que $R_9$,

ainsi que ses sels d'addition d'acides cosmétiquement ou pharmaceutiquement acceptables.

**17.** Composé selon la revendication l6, cractérisé par le fait qu'il est choisi parmi :
- le méthyl-2 amino-4 éthoxy-6 pyrimidine oxyde-3 ;
- le méthyl-2 amino-4 (diméthyl-2,4 phényl)oxy-6 pyrimidine oxyde-3 ;
- le méthyl-2 amino-4 (méthyl-1)éthyloxy-6 pyrimidine oxyde-3 ;
- le méthyl-2 amino-4 butoxy-6 pyrimidine oxyde-3 ;
- le méthyl-2 éthoxycarbonylamino-4 pipéridino-6 pyrimidine oxyde-3 ;
- le diméthyl-2,4 pipéridino-6 pyrimidine oxyde-3;
- le sel interne de l'hydroxyde de diméthyl-2,4 pipéridino-6 sulfoxy-3 pyrimidinium.

**18.** Composé, caractérisé par le fait qu'il répond à la formule suivante :

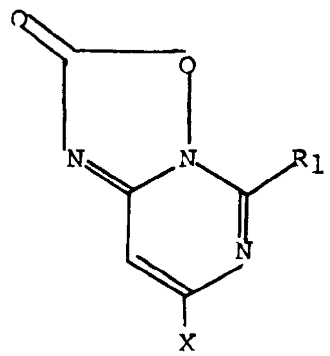

(VIII)

dans laquelle $R_1$ désigne un atome d'hydrogène ou un radical alkyle linéaire en $C_1$-$C_8$;

X désigne :

(i) un groupement

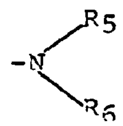

dans lequel :

$R_5$ et $R_6$, identiques ou différents, désignent un atome d'hydrogène, un groupe alkyle en $C_1$-$C_{12}$, linéaire ou ramifié, pouvant être substitué par un ou plusieurs atomes d'halogène, un groupe alcényle linéaire en $C_2$-$C_{12}$, un groupe cycloalkyle en $C_3$-$C_{10}$ ou un groupe aryle ou aralkyle répondant à la formule :

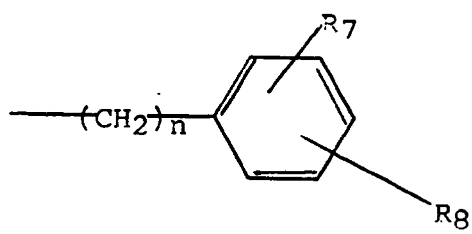

où

n vaut O à 4;

$R_7$ et/ou $R_8$, indépendamment l'un de l'autre, désignent un atome d'hydrogène, un groupement alkyle ou alcoxy inférieur en $C_1$-$C_6$ ou un radical trifluorométhyle;

$R_5$ et $R_6$ peuvent former avec l'atome d'azote auquel ils sont reliés, un hétérocycle saturé ou insaturé, choisi parmi les groupes suivants : aziridino, azétidino, pyrrolidino, pipéridino, hexaméthylèneimino, heptaméthylèneimino, octaméthylèneimino, tétrahydropyridino, dihydropyridino, pyrrole, pyrrazole, imidazole, triazole, alkyl-4 pipérazino, morpholino, thiamorpholino;

(ii) un groupement -$OR_9$ dans lequel $R_9$ désigne un radical alkyle en $C_1$-$C_{12}$, linéaire ou ramifié, pouvant être substitué par un ou plusieurs atomes d'halogène, un radical alcényle en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un radical alkyle en $C_1$-$C_6$, un radical alcoxy en $C_1$-$C_6$, un atome d'halogène ou un radical trifluorométhyle;

(iii) un groupement -$SR_{10}$ dans lequel $R_{10}$ a la même signification que $R_9$ indiqué ci-dessus,

ainsi que ses sels d'addition d'acides cosmétiquement ou pharmaceutiquement acceptables.

**19.** Composition pharmaceutique ou cosmétique destinée à être utilisée en application topique pour freiner la chute des cheveux et pour induire et stimuler leur croissance, caractérisée par le fait qu'elle contient dans un milieu physiologiquement acceptable, au moins un composé répondant à la formule (VIII) définie dans la revendication 17.

**20.** Utilisation des composés répondant à la formule générale (I) :

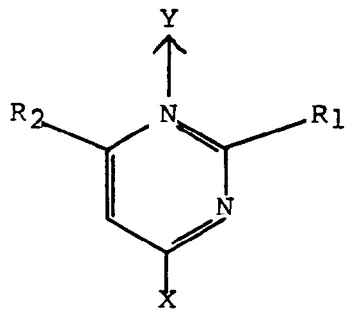

(I)

dans laquelle $R_1$, $R_2$, X et Y ont la signification indiquée dans la revendication 1, ou de leurs sels d'addition d'acides pharmaceutiquement acceptables, pour la préparation d'un médicament destiné au traitement thérapeutique de l'alopécie, de la pelade, de la chute des cheveux et des dermatites desquamantes.

**21.** Procédé de traitement cosmétique des cheveux ou du cuir chevelu, caractérisé par le fait que l'on applique une composition telle que définie dans les revendications 1 à 15 et 18.

**Patentansprüche**

**1.** Zusammensetzung zur Verwendung zum Verlangsamen des Haarausfalls sowie zum Auslösen und Anregen des Haarwachstums,
dadurch gekennzeichnet,
daß sie in einem physiologisch akzeptablen Medium mindestens eine Verbindung, die der folgenden Formel (I) entspricht

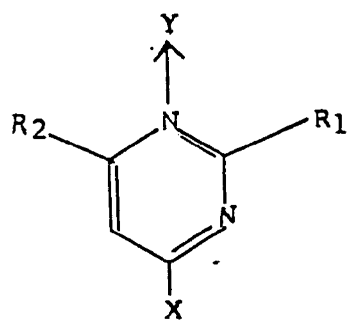

(I)

in der

- $R_1$ ein Wasserstoffatom oder einen geradkettigen gesättigten $(C_1\text{-}C_8)$-Alkylrest bezeichnet,
- $R_2$ einen geradkettigen gesättigten $(C_1\text{-}C_8)$-Alkylrest oder eine $-NHR_3$-Gruppierung bezeichnet, in welcher $R_3$ ein Wasserstoffatom oder eine $-COOR_4$-Gruppe bedeutet, in der $R_4$ einen geradkettigen linearen $(C_1\text{-}C_4)$-Rest darstellt;
- X bezeichnet:

(i) eine

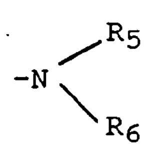 -Gruppe,

in der
$R_5$ und $R_6$ gleich oder verschieden sind und ein Wasserstoffatom; eine geradkettige oder verzweigte

($C_1$-$C_{12}$)-Alkylgruppe, die durch eine oder mehrere Halogenatome subsituiert sein kann; eine geradkettige ($C_2$-$C_{12}$)-Alkenylgruppe, eine ($C_3$-$C_{10}$)-Cycloalkylgruppe oder eine Aryl- oder Aralkylgruppe der folgenden Formel bezeichnen:

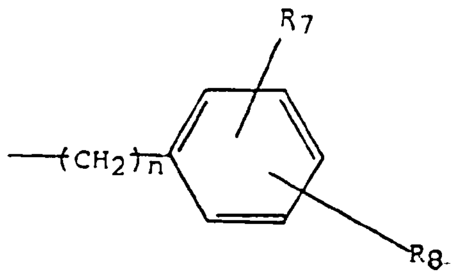

worin n gleich 0 bis 4 ist; und

$R_7$ und/oder $R_8$ unabhängig voneinander ein Wasserstoffatom, eine niedrige Alkyl- oder Alkoxygruppe mit 1-6 Kohlenstoffatomen oder einen Trifluormethylrest bezeichne(t)n;

$R_5$ und $R_8$ mit dem Stickstoffatom, an das sie gebunden sind, eine gesättigte oder ungesättigte heterocyclische Gruppe, ausgewählt aus den folgenden Gruppen: der Aziridino-, Azetidino-, Pyrrolidino-, Piperidino-, Hexamethylenimino-, Heptamethylenimino-, Oktamethylenimino-, Tetrahydropyridino-, Dihydropyridino-, Pyrrol-, Pyrrazol-, Imidazol-, Triazol-, 4-Alkyl-piperazino-, Morpholino-, Thiomorpholino-Gruppe, bilden können;

(ii) eine Gruppierung -$OR_9$,

in der $R_9$ einen geradkettigen oder verzweigten ($C_1$-$C_{12}$)-Alkylrest, der durch ein oder mehrere Halogenatome substituiert sein kann; einen ($C_2$-$C_{12}$)-Alkenylrest; einen ($C_3$-$C_{10}$)-Cycloalkylrest; einen ($C_7$-$C_{12}$)-Aralkylrest; einen Phenylrest, der gegebenenfalls durch zwei Gruppierungen substituiert ist, die unabhängig voneinander einen ($C_1$-$C_6$)-Alkylrest, einen ($C_1$-$C_6$)-Alkoxyrest, ein Halogenatom oder einen Trifluormethylrest darstellen, bezeichnen;

(iii) eine Gruppierung -$SR_{10}$, in der $R_{10}$ die gleiche Bedeutung hat wie $R_9$, das oben definiert wurde;

- Y ein Sauerstoffatom oder eine Gruppierung -$OSO_3^{\ominus}$ bezeichnet;

oder eines ihrer physiologisch akzeptablen Additionssalze mit Säuren enthält.

**2.** Zusammensetzung nach Anspruch 1,
dadurch gekennzeichnet,
daß die Verbindung der Formel (I) aus denen ausgewählt ist, für die:
$R_1$ und $R_2$ einen Methylrest bezeichnen, und X die Piperidinogruppe bezeichnet; oder
$R_1$ Methyl bezeichnet und $R_2$ -$NHR_3$ bezeichnet, in der $R_3$ die gleiche Bedeutung hat, wie im Anspruch 1 angegeben ist, und X eine Dimethylamino-, Diethylamino-, n-Butylamino-, Piperidino-, Morpholino-, 4-Methyl-piperazino-, Benzylamino- oder Anilino-Gruppe bezeichnet.

**3.** Zusammensetzung nach Anspruch 1,
dadurch gekennzeichnet,
daß die Verbindung der Formel (I) aus denen ausgewählt ist, für die
$R_1$ und $R_2$ Methyl bezeichnen, und X die Ethoxygruppe darstellt; oder
$R_1$ Methyl bezeichnet und $R_2$ -$NHR_3$ bezeichnet, wobei $R_3$ die gleiche Bedeutung hat, wie in Formel (I) angegeben ist, und X die Ethoxy-, Butoxy-, 1-Methyl-ethyloxy- oder 2,4-Dimethyl-phenyloxy-Gruppe bezeichnet.

**4.** Pharmazeutische oder kosmetische Zusammensetzung zur lokalen Anwendung,
dadurch gekennzeichnet,
daß sie in einem physiologisch akzeptablen Medium mindestens eine Verbindung enhält, wie sie in einem der Ansprüche 1 bis 3 definiert ist.

**5.** Zusammensetzung nach Anspruch 4,
dadurch gekennzeichnet,
daß sie im Hinblick auf ihre pharmazeutische Anwendung als Salbe, Tinktur, Creme, Pomade, Pulver, Pflaster, durchtränkter Bausch, Lösung, Emulsion, bläschenartige Dispersion, Lotion, Gel, Spray oder wasserfreie oder wäßrige Suspension vorliegt und daß sie mindestens eine Verbindung, wie sie in einem der

Ansprüche 1 bis 3 definiert ist, enthält.

6. Pharmazeutische Zusammensetzung nach Anspruch 5, dadurch gekennzeichnet, daß die Verbindung der Formel (I) in Konzentrationen zwischen 0,1 und 10 Gew.-%, bezogen auf das Gesamtgewicht der Zusammensetzung, enthalten ist.

7. Zusammensetzung zur kosmetischen Verwendung, wie sie in Anspruch 4 definiert ist, dadurch gekennzeichnet, daß sie als Lotion, Gel, Seife, Shampoo, Aerosol oder Schaum vorliegt und daß sie in einem kosmetisch akzeptablen Träger mindestens eine Verbindung, wie sie in einem der Ansprüche 1 bis 3 definiert ist, in einer Konzentration zwischen 0,01 und 5 Gew.-% enthält.

8. Zusammensetzung nach einem der Ansprüche 4 bis 7, dadurch gekennzeichnet, daß sie außerdem wasseranlagernde Mittel und Mittel gegen Seborrhöe enthält.

9. Zusammensetzung nach einem der Ansprüche 4 bis 8, dadurch gekennzeichnet, daß sie außerdem Mittel enthält, die auch die Wirksamkeit der Verbindungen der Formel (I) hinsichtlich der Verhinderung und/oder dem Verlangsamen von Haarausfall noch verbessern.

10. Zusammensetzung nach Anspruch 9, dadurch gekennzeichnet, daß sie als Mittel, die die Wirksamkeit zur Verhinderung und/oder Verlangsamung von Haarausfall noch verbessern, Nicotinsäureester, steroidische oder nichtsteroidische anti-inflammatorische Mittel, Retinoide, antibakterielle Mittel, Calciumantagonisten, Hormone, anti-androgene Mittel, Fänger für HO-Radikale enthält.

11. Zusammensetzung nach Anspruch 9, dadurch gekennzeichnet, daß sie als Mittel, die die Wirksamkeit zur Verhinderung und/oder zum Verlangsamen von Haarausfall noch verbessern, Verbindungen, ausgewählt aus Diazoxid, Spiroxazon, Phospholipiden, Linolsäure, Linolensäure, Salicylsäure und ihren Derivaten, Hydroxycarbonsäuren oder Ketocarbonsäuren, ihren Estern und ihren entsprechenden Salzen, Anthralin, Karotinoiden, 5,8,11,14-Eikosatetraensäure, ihren Estern und Amiden, 5,8,11-Eikosatriensäure, ihren Estern und Amiden, enthält.

12. Zusammensetzung nach einem der Ansprüche 4 bis 11, dadurch gekennzeichnet, daß sie auch oberflächenaktive Mittel, ausgewählt aus den nicht-ionischen und amphoteren oberflächenaktiven Mitteln, enthält.

13. Zusammensetzung nach einem der Ansprüche 4 bis 12, dadurch gekennzeichnet, daß das physiologisch akzeptable Medium aus Wasser, einer Mischung aus Wasser und einem oder mehreren organischen Lösungsmittel(n) oder aus einer Mischung organischer Lösungsmittel besteht, wobei die organischen Lösungsmittel pharmazeutisch oder kosmetisch akzeptabel sind.

14. Zusammensetzung nach Anspruch 13, dadurch gekennzeichnet, daß die Lösungsmittel aus den niederen Alkoholen mit 1 bis 4 Kohlenstoffatomen, den Alkylenglykolen und den Alkylethern von Mono- und Dialkylenglykol ausgewählt sind.

15. Zusammensetzung nach einem der Ansprüche 4 bis 14, dadurch gekennzeichnet, daß das physiologisch akzeptable Medium durch Verdickungsmittel und/oder Geliermittel verdickt ist und Konvervierungsmittel, Stabilisatoren, den pH-Wert regulierende Mittel, den osmotischen Druck verändernde Mittel, Emulgatoren, Filter für UV-A und UV-B, Antioxidantien enthält.

**16.** Verbindung, dadurch gekennzeichnet, daß sie der folgenden Formel entspricht:

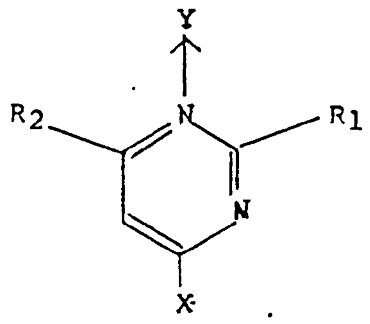

(I')

in der

- $R_1$ ein Wasserstoffatom oder einen geradkettigen gesättigten ($C_1$-$C_8$)-Alkylrest bezeichnet;
- $R_2$ einen geradkettigen, gesättigten ($C_1$-$C_8$)-Alkylrest, eine Gruppierung -$NHR_3$, in der $R_3$ ein Wasserstoffatom darstellt, oder die Gruppe -$COOR_4$, in der $R_4$ einen geradkettigen ($C_1$-$C_4$)-Alkylrest darstellt, bezeichnet;
- Y ein Sauerstoffatom oder die Gruppierung -$OSO_3^{\ominus}$ bezeichnet;
- X bezeichnet:

(i) eine Gruppierung

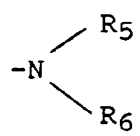

in der

$R_5$ und $R_6$, gleich oder verschieden sind, ein Wasserstoffatom; eine geradkettige oder verzweigte ($C_1$-$C_{12}$)-Alkylgruppe, die durch ein oder mehrere Halogenatome subsituiert sein kann; eine geradkettige ($C_2$-$C_{12}$)-Alkenylgruppe; eine ($C_3$-$C_{10}$)-Cycloalkylgruppe oder eine Aryl- oder Aralkylgruppe der Formel bezeichnen:

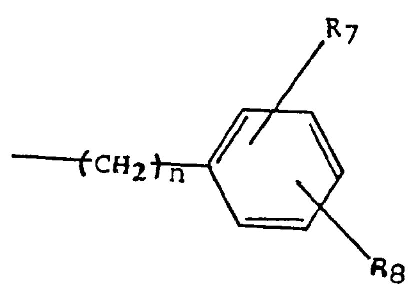

worin n gleich 0 bis 4 ist;

$R_7$ und/oder $R_8$ unabhängig voneinander ein Wasserstoffatom, eine niedrigere Alkyl- oder Alkoxygruppe mit 1 bis 6 Kohlenstoffatomen oder einen Trifluormethylrest bezeichnen;

$R_5$ und $R_6$ mit dem Stickstoffatom, an das sie gebunden sind, eine gesättigte oder ungesättigte heterocyclische Gruppe, ausgewählt aus den folgenden Gruppen: der Aziridino-, Azetidino-, Pyrrolidino-, Piperidino-, Hexamethylenimino-, Heptamethylenimino-, Oktamethylenimino-, Tetrahydropyridino-, Dihydropyridino-, Pyrrol-, Pyrrazol-, Imidazol-, Triazol-, 4-Alkylpiperazino-, Morpholino-, Thiomorpholino-Gruppe, bilden können; unter der Voraussetzung, daß

- wenn Y ein Sauerstoffatom bezeichnet, $R_1$ und $R_2$, unabhängig voneinander, einen geradkettigen, gesättigten ($C_1$-$C_8$)-Alkylrest darstellen und nicht gleichzeitig den Methylrest bezeichnen, wenn X Dimethylamino bezeichnet;

- wenn Y die Gruppe $-OSO_3^{\ominus}$ bezeichnet, $R_2$ nicht $-NH_2$ ist;

(ii) eine Gruppierung $-OR_9$, in welcher $R_9$ einen geradkettigen oder verzweigten $(C_1-C_{12})$-Alkylrest, der durch ein oder mehrere Halogenatome substituiert ist, einen $(C_2-C_{12})$-Alkenylrest, einen $(C_3-C_{10})$-Cycloalkylrest, einen $(C_7-C_{12})$-Aralkylrest bezeichnet; unter der Voraussetzung, daß

- wenn Y ein Sauerstoffatom bezeichnet, $R_2$ eine Gruppierung $-NHR_3$, wie sie oben definiert ist, bezeichnet;

(iii) eine Gruppe $-SR_{10}$, in der $R_{10}$ die gleiche Bedeutung wie $R_9$ hat,

sowie ihre kosmetisch oder pharmazeutisch akzeptablen Additionssalze mit Säuren.

**17.** Verbindung nach Anspruch 16,
dadurch gekennzeichnet,
daß sie unter folgenden Verbindungen ausgewählt ist:

- 2-Methyl-4-amino-6-ethoxy-pyrimidin-3-oxid;
- 2-Methyl-4-amino-6-(2,4-dimethyl-phenyl)-oxy-pyrimidin-3-oxid;
- 2-Methyl-4-amino-6-(1-methyl)-ethyloxy-pyrimidin-3-oxid;
- 2-Methyl-4-amino-6-butoxy-pyrimidin-3-oxid;
- 2-Methyl-4-ethoxycarbonylamino-6-piperidino-pyrimidin-3-oxid;
- 2,4-Dimethyl-6-piperidino-pyrimidin-3-oxid;
- das innere Salz von 2,4-Dimethyl-6-piperidino-3-sulfooxy-pyridiniumhydroxid.

**18.** Verbindung,
dadurch gekennzeichnet,
daß sie der folgenden Formel entspricht

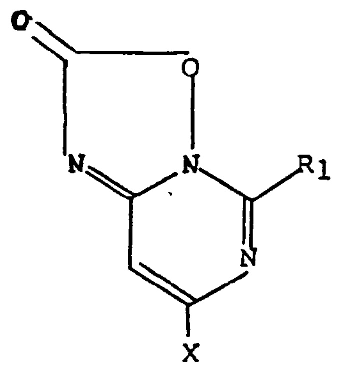

(VIII)

in welcher $R_1$ ein Wasserstoffatom oder einen geradkettigen $(C_1-C_8)$-Alkylrest bezeichnet;
X bezeichnet:

(i) eine Gruppierung

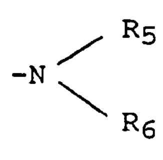

in welcher
$R_5$ und $R_6$, gleich oder verschieden sind, ein Wasserstoffatom, eine geradkettige oder verzweigte $(C_1-C_{12})$-Alkylgruppe, die durch ein oder mehrere Chloratome substituiert ist, eine geradkettige $(C_2-C_{12})$-Alkenylgruppe, eine $(C_3-C_{10})$-Cycloalkylgruppe oder eine Aryl- oder Aralkyl-Gruppe der folgenden Formel bezeichnen:

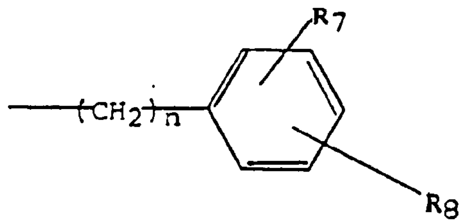

worin
n gleich 0 bis 4 ist;
$R_7$ und/oder $R_8$ unabhängig voneinander ein Wasserstoffatom, eine niedrigere Alkyl- oder Alkoxygruppe mit 1 bis 6 Kohlenstoffatomen oder einen Trifluormethylrest bezeichnen;
$R_5$ und $R_6$ mit dem Stickstoffatom, an das sie gebunden sind, eine gesättigte oder ungesättigte heterocyclische Gruppe, ausgewählt aus folgenden Gruppen: der Aziridino-, Azetidino-, Pyrrolidino-, Piperidino-, Hexamethylenimino-, Heptamethylenimino-, Oktamethylenimino-, Tetrahydropyridino-, Dihydropyridino-, Pyrrol-, Pyrrazol-, Imidazol-, Triazol-, 4-Alkyl-piperazino-, Morpholino-, Thiomorpholino-Gruppe, bilden können;
(ii) eine Gruppierung $-OR_9$, in welcher $R_9$ einen geradkettigen oder verzweigten Alkylrest mit 1 bis 12 Kohlenstoffatomen, der durch ein oder mehrere Halogenatome substituiert sein kann, einen $(C_2-C_{12})$-Alkenylrest, einen $(C_3-C_{10})$-Cycloalkylrest, einen $(C_7-C_{12})$-Aralkylrest, einen Phenylrest, der gegebenenfalls durch ein oder zwei Gruppierungen, die unabhängig voneinander einen $(C_1-C_6)$-Alkylrest, einen $(C_1-C_6)$-Alkoxyrest, ein Halogenatom oder einen Trifluormethylrest darstellen, bezeichnet;
(iii) eine Gruppierung $-SR_{10}$, in welcher $R_{10}$ die gleiche Bedeutung wie $R_9$, die oben angegeben ist, hat,
sowie ihre kosmetisch oder pharmazeutisch akzeptablen Additionssalze mit Säuren.

**19.** Pharmazeutische oder kosmetische Zusammensetzung zur lokalen Anwendung zum Verlangsamen des Haarausfalls und zum Auslösen und Anregen des Haarwachstums,
dadurch gekennzeichnet,
daß sie in einem physiologisch akzeptablen Medium mindestens eine Verbindung enthält, die der in Anspruch 17 definierten Formel (VIII) entspricht.

**20.** Verwendung von Verbindungen, die der allgemeinen Formel (I)

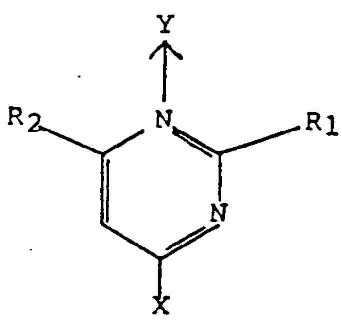

(I)

in welcher $R_1$, $R_2$, X und Y die in Anspruch 1 angegebene Bedeutung haben, entsprechen, sowie ihrer pharmazeutisch akzeptablen Additionssalze mit Säuren zur Herstellung eines Arzneimittels zur therapeutischen Behandlung von Haarausfall und Schuppen.

**21.** Kosmetisches Behandlungsverfahren für Haare oder Kopfhaut,
dadurch gekennzeichnet,
daß man eine Zusammensetzung, wie sie in den Ansprüchen 1 bis 15 und 18 definiert ist, aufträgt.

## Revendications

**1.** Composition intended to be used for retarding hair loss and for inducing and stimulating hair growth, characterised in that it contains, in a physiologically acceptable medium, at least one compound corresponding to the following formula (I):

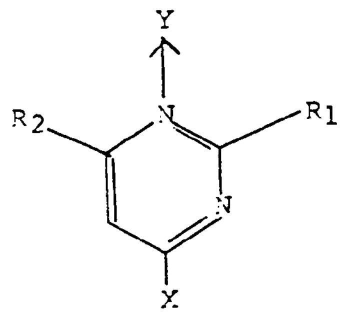

(I)

in which:

$R_1$ denotes a hydrogen atom or a $C_1$-$C_8$ saturated straight-chain alkyl radical;

$R_2$ denotes a $C_1$-$C_8$ saturated straight-chain alkyl radical, an -$NHR_3$ group in which $R_3$ denotes a hydrogen atom, or the group -$COOR_4$, where $R_4$ represents a $C_1$-$C_4$ straight-chain alkyl radical;

X denotes:

(i) a

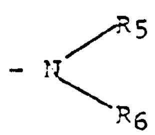

group

in which:

$R_5$ and $R_6$, which may be identical or different, denote a hydrogen atom, a straight-chain or branched $C_1$-$C_{12}$ alkyl group, which may be substituted by one or more halogen atoms, a $C_2$-$C_{12}$ straight-chain alkenyl group, a $C_3$-$C_{10}$ cycloalkyl group or an aryl or aralkyl group corresponding to the formula:

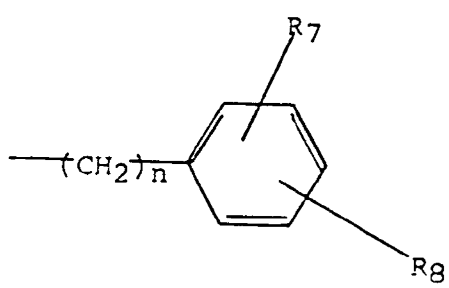

where

n ranges from 0 to 4; and

$R_7$ and/or $R_8$, independently of one another, denote a hydrogen atom, a $C_1$-$C_6$ lower alkyl or alkoxy group or a trifluoromethyl radical; and

$R_5$ and $R_6$, together with the nitrogen atom to which they are bonded, may form a saturated or unsaturated heterocycle chosen from the following groups: aziridino, azetidino, pyrrolidino, piperidino, hexamethyleneimino, heptamethyleneimino, octamethyleneimino, tetrahydropyridino, dihydropyridino, pyrrole, pyrrazole, imidazole, triazole, 4-alkylpiperazino, morpholino and thiamorpholino;

(ii) an -$OR_9$ group, in which $R_9$ denotes a straight-chain or branched $C_1$-$C_{12}$ alkyl radical, which may be substituted by one or more halogen atoms, a $C_2$-$C_{12}$ alkenyl radical, a $C_3$-$C_{10}$ cycloalkyl radical, a $C_7$-$C_{12}$ aralkyl radical or a phenyl radical, which may optionally be substituted by one or two groups which, independently of one another, denote a $C_1$-$C_6$ alkyl radical, a $C_1$-$C_6$ alkoxy radical, a halogen atom or a trifluoromethyl radical; or

(iii) an -$SR_{10}$ group, in which $R_{10}$ has the same meaning as $R_9$ defined above; and

Y denotes an oxygen atom or an $-OSO_3^{\ominus}$ group,
or its physiologically acceptable acid addition salts.

2. Composition according to Claim 1, characterised in that the compound of formula (I) is chosen from those for which:
   $R_1$ and $R_2$ denote a methyl radical and X denotes the piperidino group; or
   $R_1$ denotes methyl and $R_2$ denotes $-NHR_3$, in which $R_3$ has the same meaning indicated in Claim 1, and X denotes dimethylamino, diethylamino, n-butylamino, piperidino, morpholino, 4-methylpiperazino, benzylamino or anilino.

3. Composition according to Claim 1, characterised in that the compound of formula (I) is chosen from those for which:
   $R_1$ and $R_2$ denote methyl and X represents the ethoxy group; or
   $R_1$ denotes methyl and $R_2$ denotes $-NHR_3$, in which $R_3$ has the same meaning mentioned in formula (I), and X denotes the ethoxy, butoxy, 1-methylethoxy or 2,4-dimethylphenoxy group.

4. Pharmaceutical or cosmetic composition intended to be used by topical application, characterised in that it contains, in a physiologically acceptable medium, at least one compound as defined in any one of Claims 1 to 3.

5. Composition according to Claim 4, characterised in that it is in the form of an ointment, dye, cream, pomade, powder, patch, impregnated buffer, vesicular solution, emulsion or dispersion, lotion, gel, spray or suspension, which is anhydrous or aqueous, with a view to its pharmaceutical application, and in that it contains at least one compound as defined in any one of Claims 1 to 3.

6. Pharmaceutical composition according to Claim 5, characterised in that the compound of formula (I) is present in concentrations of between 0.1 and 10% by weight relative to the total weight of the composition.

7. Composition intended to be used in cosmetics, as defined in Claim 4, characterised in that it is in the form of a lotion, gel, soap, shampoo, aerosol or foam and in that it contains, in a cosmetically acceptable excipient, at least one compound as defined in any one of Claims 1 to 3, in a concentration of between 0.01 and 5% by weight.

8. Composition according to any one of Claims 4 to 7, characterised in that it also contains hydrating agents and antiseborrheic agents.

9. Composition according to any one of Claims 4 to 8, characterised in that it also contains agents which further improve the activity of the compounds of formula (I) in respect of hair regrowth and/or retardation of hair loss.

10. Composition according to Claim 9, characterised in that it contains, as agents further improving the activity in respect of hair regrowth and/or retardation of hair loss, nicotinic acid esters, steroid or non-steroid anti-inflammatory agents, retinoids, antibacterial agents, calcium antagonists, hormones, antiandrogenic agents or compounds which capture OH radicals.

11. Composition according to Claim 9, characterised in that it contains, as compounds further improving the activity in respect of hair regrowth and/or retardation of hair loss, compounds chosen from diazoxide, spiroxazone, phospholipids, linoleic and linolenic acids, salicylic acid and its derivatives, hydroxycarboxylic or ketocarboxylic acids, their esters, the lactones and their corresponding salts, anthralin, carotenoids, 5,8,11,14-eicosatetrainoic and 5,8,11-eicosatriinoic acids and their esters and amides.

12. Composition according to any one of Claims 4 to 11, characterised in that it also contains surfactants chosen from nonionic and amphoteric surfactants.

13. Composition according to any one of Claims 4 to 12, characterised in that the physiologically acceptable medium consists of water, a mixture of water and one or more organic solvents or a mixture of organic solvents, the organic solvents being pharmaceutically or cosmetically acceptable.

14. Composition according to Claim 13, characterised in that the solvents are chosen from $C_1$-$C_4$ lower alcohols, alkylene glycols and monoalkylene and dialkylene glycol alkyl ethers.

15. Composition according to any one of Claims 4 to 14, characterised in that the physiologically acceptable medium is thickened using thickeners and/or gelling agents and contains preservatives, stabilisers, pH regulators, agents which modify the osmotic pressure, emulsifiers, UVA and UVB filters and antioxidants.

16. Compound, characterised in that it corresponds to the following formula:

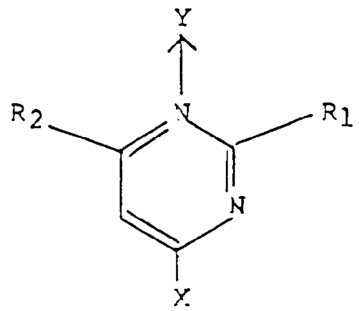

(I')

in which:

$R_1$ denotes a hydrogen atom or a $C_1$-$C_8$ saturated straight-chain alkyl radical;

$R_2$ denotes a $C_1$-$C_8$ saturated straight-chain alkyl radical, an $-NHR_3$ group in which $R_3$ denotes a hydrogen atom, or the group $-COOR_4$, where $R_4$ represents a $C_1$-$C_4$ straight-chain alkyl radical;

Y denotes an oxygen atom or the $OSO_3^{\ominus}$ group;

X denotes:

(i) a

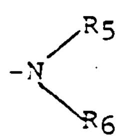

group
in which:

$R_5$ and $R_6$, which may be identical or different, denote a hydrogen atom, a straight-chain or branched C1-$C_{12}$ alkyl group, which may be substituted by one or more halogen atoms, a $C_2$-$C_{12}$ straight-chain alkenyl group, a $C_3$-$C_{10}$ cycloalkyl group or an aryl or aralkyl group corresponding to the formula:

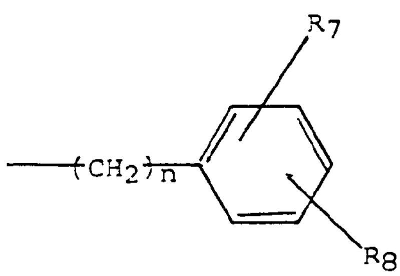

where

n ranges from 0 to 4; and

$R_7$ and/or $R_8$, independently of one another, denote a hydrogen atom, a $C_1$-$C_6$ lower alkyl or alkoxy group or a trifluoromethyl radical; and

$R_5$ and $R_6$, together with the nitrogen atom to which they are bonded, may form a saturated or unsaturated heterocycle chosen from the following groups: aziridino, azetidino, pyrrolidino, piperidino, hexamethyleneimino, heptamethyleneimino, octamethyleneimino, tetrahydropyridino, dihydropyridino, pyrrole, pyrrazole, imidazole, triazole, 4-alkylpiperazino, morpholino and thiamorpholino; with the proviso that:

- when Y denotes an oxygen atom, $R_1$ and $R_2$ denote, independently of one another, a $C_1$-$C_8$ sa-

turated straight-chain alkyl radical and do not simultaneously denote the methyl radical when X denotes dimethylamino; and

- when Y denotes the $OSO_3^{\ominus}$ group, $R_2$ does not denote $-NH_2$;

(ii) an $-OR_9$ group, in which $R_9$ denotes a straight-chain or branched $C_1$-$C_{12}$ alkyl radical, which may be substituted by one or more halogen atoms, a $C_2$-$C_{12}$ alkenyl radical, a $C_3$-$C_{10}$ cycloalkyl radical, a $C_7$-$C_{12}$ aralkyl radical, with the proviso that:

- when Y denotes an oxygen atom, $R_2$ denotes an $-NHR_3$ group as defined above;

(iii) an $-SR_{10}$ group, in which $R_{10}$ has the same meaning as $R_9$,

as well as its cosmetically or pharmaceutically acceptable acid addition salts.

**17.** Compound according to Claim 16, characterised in that it is chosen from:

- 2-methyl-4-amino-6-ethoxypyrimidine 3-oxide;
- 2-methyl-4-amino-6-(2,4-dimethylphenyl)oxypyrimidine 3-oxide;
- 2-methyl-4-amino-6-(1-methyl)ethoxypyrimidine 3-oxide;
- 2-methyl-4-amino-6-butoxypyrimidine 3-oxide;
- 2-methyl-4-ethoxycarbonylamino-6-piperidinopyrimidine 3-oxide;
- 2,4-dimethyl-6-piperidinopyrimidine 3-oxide; and
- the inner salt of 2,4-dimethyl-6-piperidino-3-sulphoxypyrimidinium hydroxide.

**18.** Compound, characterised in that it corresponds to the following formula:

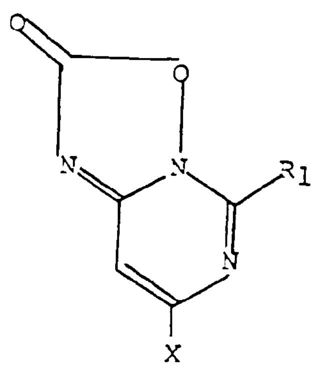

(VIII)

in which $R_1$ denotes a hydrogen atom or a $C_1$-$C_8$ straight-chain alkyl radical;

X denotes:

(i) a

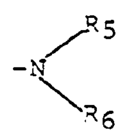

group

in which:

$R_5$ and $R_6$, which may be identical or different, denote a hydrogen atom, a straight-chain or branched $C_1$-$C_{12}$ alkyl group, which may be substituted by one or more halogen atoms, a $C_2$-$C_{12}$ straight-chain alkenyl group, a $C_3$-$C_{10}$ cycloalkyl group or an aryl or aralkyl group corresponding to the formula:

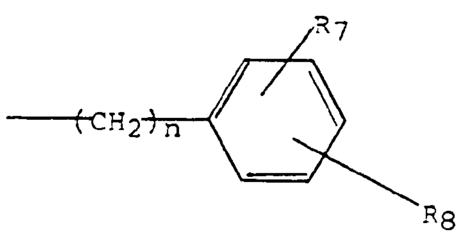

where

n ranges from 0 to 4; and

$R_7$ and/or $R_8$, independently of one another, denote a hydrogen atom, a $C_1$-$C_6$ lower alkyl or alkoxy group or a trifluoromethyl radical; and

$R_5$ and $R_6$, together with the nitrogen atom to which they are bonded, may form a saturated or unsaturated heterocycle chosen from the following groups: aziridino, azetidino, pyrrolidino, piperidino, hexamethyleneimino, heptamethyleneimino, octamethyleneimino, tetrahydropyridino, dihydropyridino, pyrrole, pyrrazole, imidazole, triazole, 4-alkylpiperazino, morpholino and thiamorpholino;

(ii) an $-OR_9$ group, in which $R_9$ denotes a straight-chain or branched $C_1$-$C_{12}$ alkyl radical, which may be substituted by one or more halogen atoms, a $C_2$-$C_{12}$ alkenyl radical, a $C_3$-$C_{10}$ cycloalkyl radical, a $C_7$-$C_{12}$ aralkyl radical or a phenyl radical, which may optionally be substituted by one or two groups which, independently of one another, denote a $C_1$-$C_6$ alkyl radical, a $C_1$-$C_6$ alkoxy radical, a halogen atom or a trifluoromethyl radical; or

(iii) an $-SR_{10}$ group, in which $R_{10}$ has the same meaning as $R_9$ defined above;

as well as its cosmetically or pharmaceutically acceptable acid addition salts.

**19.** Pharmaceutical or cosmetic composition intended to be used, by topical application, for retarding hair loss and for inducing and stimulating hair growth, characterised in that it contains, in a physiologically acceptable medium, at least one compound corresponding to the formula (VIII) defined in Claim 17.

**20.** Use of compounds corresponding to the general formula (I):

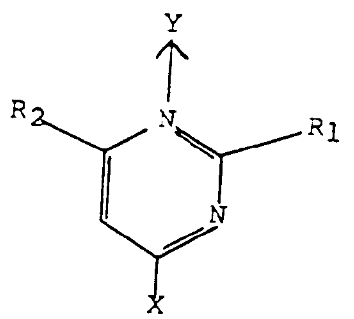

(I)

in which $R_1$, $R_2$, X and Y have the meaning indicated in Claim 1, or their pharmaceutically acceptable acid addition salts, for the preparation of a medicament intended for the therapeutic treatment of alopecia, pelade, hair loss and desquamative dermatites.

**21.** Process for the cosmetic treatment of hair or the scalp, characterised in that a composition as defined in Claims 1 to 15 and 18 is applied.